# EUROPEAN PATENT APPLICATION

(11) **EP 2 014 648 A1**
(43) Date of publication of application: **14.01.2009**
(21) Application number: 06767863.1
(22) Date of filing: 28.06.2006
(51) Int. Cl.: C07D 209/52

(54) **PROCESS FOR PRODUCING POLYCYCLIC PROLINE DERIVATIVE OR ACID ADDUCT SALT THEREOF**

(30) Priority: 17.04.2006 JP 2006113074
(71) Applicant: Sumitomo Chemical Company, Limited, Tokyo 104-8260 (JP)
(72) Inventor: HIRATA, Norihiko, Suita-shi, Osaka 565-0836 (JP); UEMURA, Toshitsugi, Toyonaka-shi, Osaka 561-0802 (JP); USHIO, Hideki, Ibaraki-shi, Osaka 567-0841 (JP)
(74) Representative: Duckworth, Timothy John
(86) International application number: PCT/JP2006/313355
(87) International publication number: WO 2007/122744

(57) **Abstract**

A method of producing a proline derivative of the following formula (1) or an acid addition salt thereof, comprising the following four steps A to D: 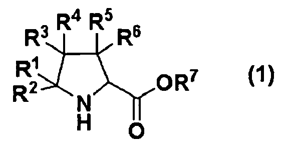 [wherein, any two of R¹, R², R³, R⁴, R⁵ and R⁶ are connected to form an optionally substituted polymethylene group having 1 to 4 carbon atoms, and R⁷ represents an optionally substituted linear alkyl group having 1 to 10 carbon atoms, branched alkyl group having 2 to 10 carbon atoms, linear alkenyl group having 2 to 10 carbon atoms, branched alkenyl group having 3 to 10 carbon atoms or aralkyl group having 7 to 20 carbon atoms.]

(Step A)

A step of reacting N-protected pyrrolidinones of the following formula (2) with a reducing agent, to produce N-protected pyrrolidinols of the following formula (3):

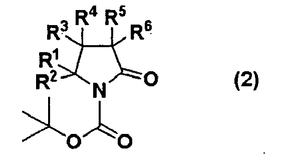

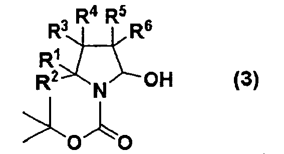

(Step B)

A step of reacting the N-protected pyrrolidinols of the formula (3) obtained in the step A with a cyanodizing agent, to produce N-protected cyanopyrrolidines of the following formula (4):

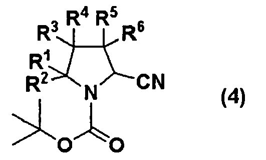

(Step C)

A step of reacting the N-protected cyanopyrrolidines of the formula (4) obtained in the step B with alcohols and a base, to obtain imidates of the following formula (5), and treating the imidates with an acid, to produce N-protected prolines of the following formula (6):

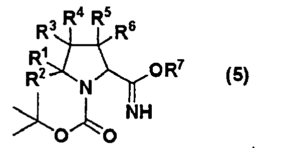

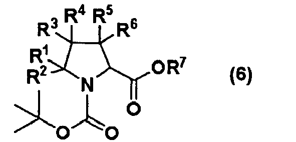

(Step D)

A step of treating the N-protected prolines of the formula (6) obtained in the step C with an acid, to produce a proline derivative of the above-described formula (1) or an acid addition salt thereof.

## Description

### Technical Field

The present invention relates to a method of producing a polycyclic proline derivative or an acid addition salt thereof.

### Background Art

Conventionally, as a method for producing a polycyclic proline derivative, the following method (see, WO-2004113295) and the like are known.

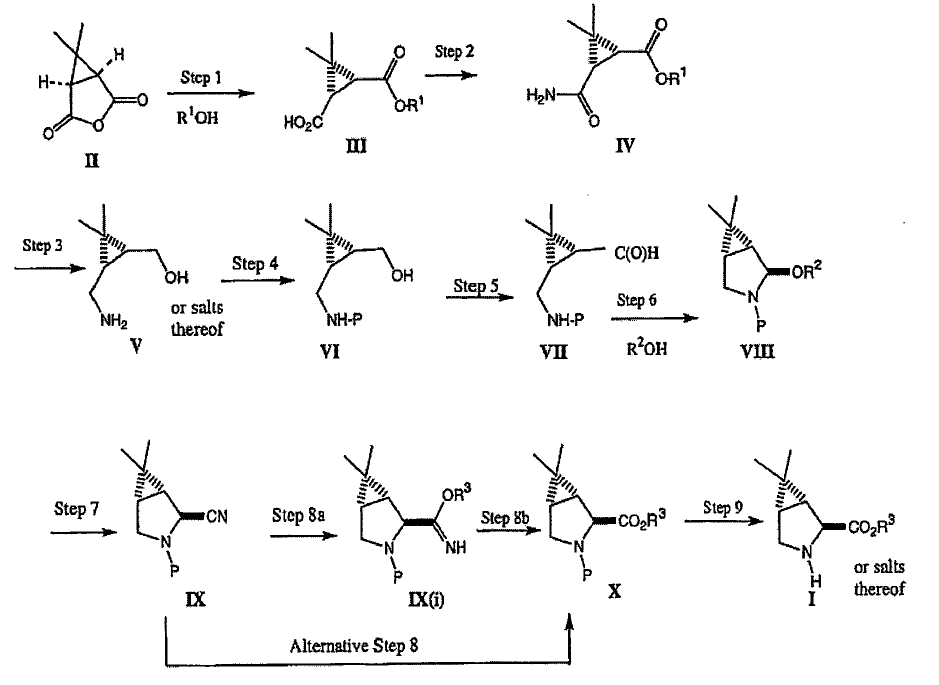

In the above-described method, however, a multi-stage complicated process including as many as 9 steps or more is necessary for production of a final compound I from a raw material compound II, additionally, the raw material compound II is a meso form compound, that is, it is necessary to carry out further an optical-activation treatment in the step I for obtaining the final compound I as an optically active body, thus, the above-described method is not recognized as an industrially simple and advantageous method.

The present inventors have investigated to find a method of producing a polycyclic proline derivative having little of the problems as described above, and found that a polycyclic proline derivative can be produced simply and industrially advantageously by using pyrrolidinones as a raw material.

### Disclosure of the Invention

The present invention has an object of providing a simple and industrially advantageous method of producing a polycyclic proline derivative or an acid addition salt thereof.

That is, the present invention provides the following [1] to [26].

[1]. A method of producing a proline derivative of the following formula (1) or an acid addition salt thereof, comprising the following four steps A to D: 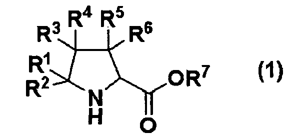 [wherein, any two of R¹, R², R³, R⁴, R⁵ and R⁶ are connected to form an optionally substituted polymethylene group having 1 to 4 carbon atoms, one or no-mutually-adjacent two methylene groups constituting the polymethylene group may be substituted by an oxygen atom, one or two ethylene groups constituting the polymethylene group may be substituted by a vinylene group, no-mutually adjacent two methylene groups constituting the polymethylene group may be mutually connected via an oxygen atom, methylene group, ethylene group or vinylene group,

R¹, R², R³, R⁴, R⁵ and R⁶ not constituting the above-described polymethylene group represent each independently a hydrogen atom, halogen atom, cyano group, optionally substituted linear, branched or cyclic alkyl group having 1 to 10 carbon atoms, optionally substituted linear, branched or cyclic alkenyl group having 2 to 10 carbon atoms, optionally substituted aryl group having 6 to 20 carbon atoms, optionally substituted amino group, -ORₐ group or - SR_{b} group, Rₐ and R_{b} represent each independently a hydrogen atom, alkylcarbonyl group having 2 to 10 carbon atoms, arylcarbonyl group having 7 to 20 carbon atoms, aralkyl group having 7 to 20 carbon atoms, alkoxyalkyl group having 2 to 10 carbon atoms, trialkylsilyl group having 3 to 10 carbon atoms, alkyl group having 1 to 10 carbon atoms or aryl group having 6 to 20 carbon atoms,

R⁷ represents an optionally substituted linear alkyl group having 1 to 10 carbon atoms, branched alkyl group having 2 to 10 carbon atoms, linear alkenyl group having 2 to 10 carbon atoms, branched alkenyl group having 3 to 10 carbon atoms or aralkyl group having 7 to 20 carbon atoms.]

### (Step A)

A step of reacting N-protected pyrrolidinones of the following formula (2) with a reducing agent, to produce N-protected pyrrolidinols of the following formula (3): 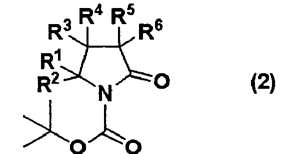 [wherein, R¹, R², R³, R⁴, R⁵ and R⁶ represent the same meanings as described above.] 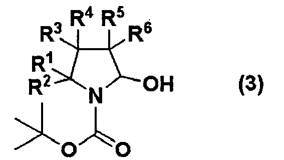 [wherein, R¹, R², R³, R⁴, R⁵ and R⁶ represent the same meanings as described above.]

### (Step B)

A step of reacting the N-protected pyrrolidinols of the formula (3) obtained in the step A with a cyanodizing agent, to produce N-protected cyanopyrrolidines of the following formula (4): 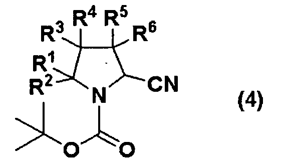 [wherein, R¹, R², R³, R⁴, R⁵ and R⁶ represent the same meanings as described above.]

### (Step C)

A step of reacting the N-protected cyanopyrrolidines of the formula (4) obtained in the step B with alcohols and a base, to obtain imidates of the following formula (5), and treating the imidates with an acid, to produce N-protected prolines of the following formula (6): 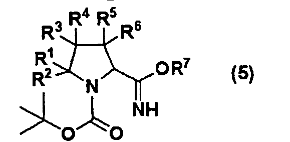 [wherein, R¹, R², R³, R⁴, R⁵, R⁶ and R⁷ represent the same meanings as described above.] 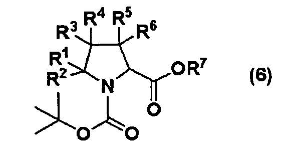 [wherein, R¹, R², R³, R⁴, R⁵, R⁶ and R⁷ represent the same meanings as described above.]

### (Step D)

A step of treating the N-protected prolines of the formula (6) obtained in the step C with an acid, to produce a proline derivative of the above-described formula (1) or an acid addition salt thereof.

[2]. A method of producing a proline derivative of the following formula (1) or an acid addition salt thereof, comprising the following three steps A, B and E: 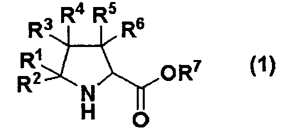 [wherein, R¹, R², R³, R⁴, R⁵, R⁶ and R⁷ represent the same meanings as described above.]

### (Step A)

A step of reacting N-protected pyrrolidinones of the following formula (2) with a reducing agent, to produce N-protected pyrrolidinols of the following formula (3): 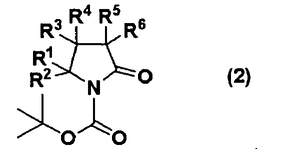 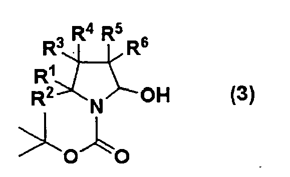 [in the formulae (2) and (3), R¹, R², R³, R⁴, R⁵ and R⁶ represent the same meanings as described above.]

### (Step B)

A step of reacting the N-protected pyrrolidinols of the formula (3) obtained in the step A with a cyanodizing agent, to produce N-protected cyanopyrrolidines of the following formula (4): 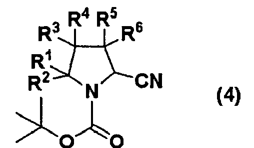 [wherein, R¹, R², R³, R⁴, R⁵ and R⁶ represent the same meanings as described above.]

### (Step E)

A step of reacting the N-protected cyanopyrrolidines of the formula (4) obtained in the Step B with alcohols and an acid, to produce a proline derivative of the formula (1) or an acid addition salt thereof.

[3]. The production method according to [1] or [2], wherein R¹ and R² represent a hydrogen atom.

[4]. The production method according to any one of [1] to [3], wherein R³ and R⁵ represent a hydrogen atom.

[5]. The production method according to any one of [1] to [4], wherein R⁴ and R⁶ are connected to form an optionally substituted polymethylene group having 1 to 4 carbon atoms.

[6]. The production method according to any one of [1] to [5], wherein R⁴ and R⁶ are connected to form a (CH₃)₂C< group.

[7]. The production method according to any one of [1] to [6], wherein R⁷ is an alkyl group having 1 to 4 carbon atoms.

[8]. The production method according to any one of [1] to [7], wherein the reducing agent to be used in the step A is lithium triethylborohydride or diisobutylaluminum hydride.

[9]. The production method according to any one of [1] to [8], wherein the cyanodizing agent to be used in the step B is trimethylsilyl cyanide.

[10]. The production method according to [9], wherein the cyanodizing agent to be used in the step B is trimethylsilyl cyanide, and a boron trifluoride complex is used together as an acid catalyst.

[11]. The production method according to [9] or [10], wherein N-protected pyrrolidinols of the formula (3) and trimethylsilyl cyanide are dropped into a solution containing a boron trifluoride complex.

[12]. The production method according to any one of [1] to [11], wherein the acid to be used in the step D and the step E is hydrogen chloride.

[13]. The production method according to any one of [1] to [12], wherein a post-treatment operation is carried out using an oxidizing agent in the step B.

[14]. N-protected pyrrolidinols of the following formula (3): 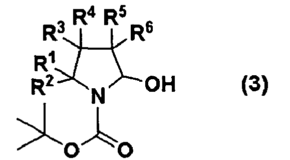 [wherein, R¹, R², R³, R⁴, R⁵ and R⁶ represent the same meanings as described above.].

[15]. The N-protected pyrrolidinols according to [14], wherein in the formula (3), R¹, R², R³ and R⁵ represent a hydrogen atom, and R⁴ and R⁶ are connected to form an optionally substituted polymethylene group having 1 to 4 carbon atoms.

[16]. 3-tert-butoxycarbonyl-6,6-dimethyl-3-azabicyclo[3.1.0]hexan-2-ol.

[17]. (1R,5S)-3-tert-butoxycarbonyl-6,6-dimethyl-3-azabicyclo[3.1.0]hexan-2-ol.

[18]. N-protected cyanopyrrolidines of the following formula (4): 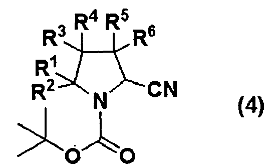 [wherein, R¹, R², R³, R⁴, R⁵ and R⁶ represent the same meanings as described above.].

[19]. The N-protected cyanopyrrolidines according to [18], wherein in the formula (4), R¹, R², R³ and R⁵ represent a hydrogen atom, and R⁴ and R⁶ are connected to form an optionally substituted polymethylene group having 1 to 4 carbon atoms.

[20]. 3-tert-butoxycarbonyl-6,6-dimethyl-3-azabicyclo[3.1.0]hexane-2-carbonitrile.

[21]. (1R,2S,5S)-3-tert-butoxycarbonyl-6,6-dimethyl-3-azabicyclo[3.1.0]hexane-2-carbonitrile.

[22]. Imidates of the following formula (5): 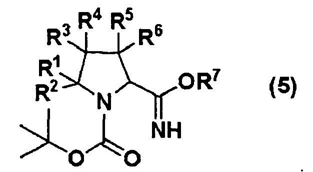 (wherein, R¹, R², R³, R⁴, R⁵, R⁶ and R⁷ represent the same meanings as described above.).

[23]. The imidates according to [22], wherein in the formula (5), R¹, R², R³ and R⁵ represent a hydrogen atom, R⁴ and R⁶ are connected to form an optionally substituted polymethylene group having 1 to 4 carbon atoms and R⁷ is an alkyl group having 1 to 4 carbon atoms.

[24]. 3-tert-butoxycarbonyl-6,6-dimethyl-3-azabicyclo[3.1.0]hexane-2-carboimidate.

[25]. Methyl 3-tert-butoxycarbonyl-6,6-dimethyl-3-azabicyclo[3.1.0]hexane-2-carboimidate.

[26]. Methyl (1R,2S,5S)-3-tert-butoxycarbonyl-6,6-dimethyl-3-azabicyclo[3.1.0]hexane-2-carboimidate.

### Modes for Carrying out the Invention

The present invention will be illustrated in detail below.

The production method of the present invention is a method containing the four steps A to D described above. First, the step A of the present invention will be illustrated.

In the N-protected pyrrolidinones of the formula (2) to be used in the present invention [hereinafter, abbreviated as N-protected pyrrolidinones (2) in some cases.], any two of R¹, R², R³, R⁴, R⁵ and R⁶ are connected to form an optionally substituted polymethylene group having 1 to 4 carbon atoms, one or no-mutually-adjacent two methylene groups constituting the polymethylene group may be substituted by an oxygen atom, one or two ethylene groups constituting the polymethylene group may be substituted by a vinylene group, no-mutually adjacent two methylene groups constituting the polymethylene group may be mutually connected via an oxygen atom, methylene group, ethylene group or vinylene group.

R¹, R², R³, R⁴, R⁵ and R⁶ not constituting the above-described polymethylene group represent each independently a hydrogen atom, halogen atom, cyano group, optionally substituted linear, branched or cyclic alkyl group having 1 to 10 carbon atoms, optionally substituted linear, branched or cyclic alkenyl group having 2 to 10 carbon atoms, optionally substituted aryl group having 6 to 20 carbon atoms, optionally substituted amino group, -ORₐ group or - SR_{b} group, Rₐ and R_{b} represent each independently a hydrogen atom, alkylcarbonyl group having 2 to 10 carbon atoms, arylcarbonyl group having 7 to 20 carbon atoms, aralkyl group having 7 to 20 carbon atoms, alkoxyalkyl group having 2 to 10 carbon atoms, trialkylsilyl group having 3 to 10 carbon atoms, alkyl group having 1 to 10 carbon atoms or aryl group having 6 to 20 carbon atoms.

Examples of the halogen atom include a chlorine atom, bromine atom, fluorine atom and iodine atom.

Examples of the optionally substituted alkyl group having 1 to 10 carbon atoms include alkyl groups having 1 to 10 carbon atoms such as a methyl group, ethyl group, n-propyl group, isopropyl group, n-butyl group, cyclohexyl group and the like; halogenated alkyl groups such as a chloromethyl group, dichloromethyl group, trichloromethyl group, fluoromethyl group, difluoromethyl group, trifluoromethyl group and the like; hydroxyalkyl groups such as a hydroxymethyl group, hydroxyethyl group and the like optionally substituted by a substituent such as a methyl group, benzyl group, phenyl group, methoxymethyl group, trimethylsilyl group and the like; aminoalkyl groups such as an aminomethyl group, aminoethyl group and the like optionally substituted by a substituent such as a methyl group, benzyl group, phenyl group, tert-butoxycarbonyl group, benzyloxycarbonyl group and the like; aralkyl groups such as a phenylmethyl group, phenylethyl group and the like optionally substituted by a substituent such as a halogen atom, alkoxy group, nitro group, cyano group, lower alkyl group, aryl group and the like; etc.

Examples of the optionally substituted alkenyl group having 2 to 10 carbon atoms include a vinyl group, ethenyl group, 1-propenyl group, 2-propenyl group, 1-butenyl group, 2-butenyl group, 3-butenyl group and the like.

Examples of the optionally substituted aryl group having 6 to 20 carbon atoms include a phenyl group, naphthyl group and the like optionally substituted by a halogen atom, alkoxy group, nitro group, cyano group or alkyl group having 1 to 6 carbon atoms.

Examples of the optionally substituted amino group include amino groups optionally substituted by a methyl group, benzyl group, tert-butoxycarbonyl group, benzyloxycarbonyl group and the like.

Examples of Rₐ of the -ORₐ group include a hydrogen atom, alkylcarbonyl groups having 2 to 10 carbon atoms such as an acetyl group and the like, arylcarbonyl groups having 7 to 20 carbon atoms such as a benzoyl group and the like, aralkyl groups having 7 to 20 carbon atoms such as a benzyl group and the like, alkoxyalkyl groups having 2 to 10 carbon atoms such as a methoxymethyl group and the like, trialkylsilyl groups having 3 to 10 carbon atoms such as a trimethylsilyl group and the like, alkyl groups having 1 to 10 carbon atoms such as a methyl group, ethyl group, n-propyl group, isopropyl group, tert-butyl group and the like, aryl groups having 6 to 20 carbon atoms such as a phenyl group and the like; etc.

Examples of R_{b} of the -SR_{b} group include a hydrogen atom, alkylcarbonyl groups having 2 to 10 carbon atoms such as an acetyl group and the like, arylcarbonyl groups having 7 to 20 carbon atoms such as a benzoyl group and the like, aralkyl groups having 7 to 20 carbon atoms such as a benzyl group and the like, alkoxyalkyl groups having 2 to 10 carbon atoms such as a methoxymethyl group and the like, trialkylsilyl groups having 3 to 10 carbon atoms such as a trimethylsilyl group and the like, alkyl groups having 1 to 10 carbon atoms such as a methyl group, ethyl group, n-propyl group, isopropyl group, tert-butyl group and the like, aryl groups having 6 to 20 carbon atoms such as a phenyl group and the like; etc.

As the substituent optionally substituted on a polymethylene group having 1 to 4 carbon atoms formed by connection of any two of R¹, R², R³, R⁴, R⁵ and R⁶ in the N-protected pyrrolidinones (2), the same substituents as the above-described substituents represented by R¹, R², R³, R⁴, R⁵ and R⁶ not constituting the polymethylene group are mentioned.

Examples of specific structures of the group formed by connection of any two of R¹, R², R³, R⁴, R⁵ and R⁶ include divalent groups of the following formulae, and the like.

-CH₂-, -(CH₂)₂-, -(CH₂)₃-, -(CH₂)₄-, (CH₃)₂C<, (Cl)₂C<, (F)₂C<, >CH(CO₂C₂H₅)

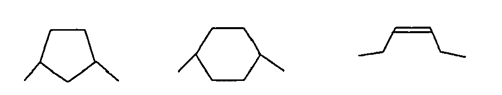

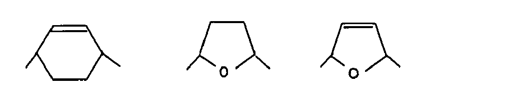

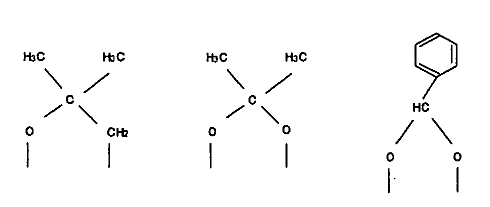

Examples of the N-protected pyrrolidinones (2) include 3-tert-butoxycarbonyl-3-azabicyclo[3.1.0]hexan-2-one, 3-tert-butoxycarbonyl-3-azabicyclo[3.2.0]heptan-2-one, 3-tert-butoxycarbonyl-3-azabicyclo[3.3.0]octan-2-one, 8-tert-butoxycarbonyl-8-azabicyclo[4.3.0]nonan-7-one, 3-tert-butoxycarbonyl-6,6-dimethyl-3-azabicyclo[3.1.0]hexan-2-one, 3-tert-butoxycarbonyl-6,6-dichloro-3-azabicyclo[3.1.0]hexan-2-one, 3-tert-butoxycarbonyl-6,6-difluoro-3-azabicyclo[3.1.0]hexan-2-one, 3-tert-butoxycarbonyl-1-phenyl-3-azabicyclo[3.1.0]hexan-2-one, 4-tert-butoxycarbonyl-4-azatricyclo[5.2.1.0^{2,6}]deca-3-one, 4-tert-butoxycarbonyl-4-azatricyclo[5.2.2.0^{2,6}]undeca-3-one, 8-tert-butoxycarbonyl-8-azabicyclo[4.3.0]nonan-3-en-7-one, 4-tert-butoxycarbonyl-4-azatricyclo[5.2.1.0^{2,6}]deca-8-en-3-one, 4-tert-butoxycarbonyl-4-azatricyclo[5.2.2.0^{2,6}]undeca-8-en-3-one, 7-tert-butoxycarbonyl-3,3-dimethyl-2,4-dioxa-7-azabicyclo[3.3.0]octan-6-one, 7-tert-butoxycarbonyl-3-phenyl-2,4-dioxa-7-azabicyclo[3.3.0]octan-6-one, 7-tert-butoxycarbonyl-3,3-dimethyl-2-oxa-7-azabicyclo[3.3.0]octan-6-one, 4-tert-butoxycarbonyl-4-aza-10-oxatricyclo[5.2.1.0^{2,6}]deca-3-one, 4-tert-butoxycarbonyl-4-aza-10-oxatricyclo[5.2.1.0^{2,6}]deca-8-en-3-one, 2-tert-butoxycarbonyl-2-azabicyclo[3,1,0]hexan-3-one, 2-tert-butoxycarbonyl-2-azabicyclo[3.3.0]octan-3-one, 7-tert-butoxycarbonyl-7-azabicyclo[4.3.0]nonan-8-one, 2-tert-butoxycarbonyl-2-azabicyclo[3.3.0]oct-7-en-3-one, 2-tert-butoxycarbonyl-2-azabicyclo[2.2.1]heptan-3-one, and optically active bodies thereof, and the like.

The N-protected pyrrolidinones (2) can be produced usually from the correspondent pyrrolidinones by a general protection method with N-Boc, for example, by a method using di-tert-butyl dicarbonate and a base, and the like.

As the N-protected pyrrolidinones (2), those prepared by other methods than the above-described methods may also be used.

The step A is a step of reacting N-protected pyrrolidinones (2) with a reducing agent to produce N-protected pyrrolidinols of the formula (3) [hereinafter, abbreviated as N-protected pyrrolidinols (3) in some cases.].

Examples of the above-described reducing agent include lithium triethylborohydride, diisobutylaluminum hydride, lithium borohydride, sodium borohydride, tetramethylammonium borohydride, tetramethylammonium triacetoxy borohydride, lithium aluminum hydride, tri-tert-butoxylithium aluminum hydride, tri-sec-butyllithium borohydride, tri-sec-butylpotassium borohydride, sodium cyano borohydride, sodium di(2-methoxyethoxy)aluminum hydride, diborane, borane complexes such as a borane-dimethyl sulfide complex and the like; etc.

Preferable reducing agents include lithium triethylborohydride and diisobutylaluminum hydride.

As these reducing agents, commercially available agents may be used, and those prepared in the reaction system may be used. Further, those in the form of solution dissolved in an organic solvent may be used.

The use amount of the above-described reducing agent is usually in the range of 0.3 to 10-mole ratio, preferably 0.5 to 5-mole ratio with respect to N-protected lactams (2).

The above-described reduction reaction is usually carried out in the presence of an organic solvent.

Examples of such an organic solvent include aliphatic hydrocarbon solvents such as hexane, heptane, cyclohexane and the like; aromatic solvents such as toluene, xylene, monochlorobenzene, dichlorobenzene and the like; ether solvents such as tetrahydrofuran, methyl tert-butyl ether, 1,4-dioxane, 1,2-dimethoxyethane and the like; halogenated hydrocarbon solvents such as dichloromethane, dichloroethane, chlorobutane and the like; alcohol solvents such as methanol, ethanol, 1-propanol, 2-propanol, 1-butanol, 2-methyl-2-propanol and the like; etc. These solvents may be used in admixture of two or more.

The use amount of the organic solvent is usually in the range of 1 to 100-weight ratio, preferably 2 to 20-weight ratio with respect to N-protected lactams (2).

The reduction reaction is usually carried out by a method of dropping a reducing agent into a solution prepared by dissolving N-protected pyrrolidinones (2) in an organic solvent, and it may also be carried out by a method of dropping a solution containing N-protected pyrrolidinones (2) into a solution containing a reducing agent.

The temperature of reduction reaction is usually in the range of -80 to 30°C, preferably -40 to 10°C.

By the reduction reaction, a reaction solution containing N-protected pyrrolidinols (3) is obtained. This reaction solution is usually subjected to a post-treatment for removal of an unreacted reducing agent and the like.

Mentioned as the post-treatment method are, for example, a method of mixing a reaction solution with water to hydrolyze a reducing agent, and if necessary, adding an organic solvent separable from water to cause liquid-partitioning, thereby, distributing inorganic components obtained by hydrolysis of the reducing agent into an aqueous layer and distributing N-protected pyrrolidinols (3) into an organic layer; and other methods.

In the case of deposition of inorganic components generated by hydrolysis of a reducing agent, these components may be removed by a filtration operation.

The water to be mixed with a reaction solution is not particularly restricted, and may be neutral water, basic aqueous solution, or acidic aqueous solution. Preferably used are aqueous solutions of inorganic bases such as alkali metal hydroxides such as sodium hydroxide, potassium hydroxide and the like, alkali metal carbonates such as sodium carbonate, potassium carbonate and the like, alkali metal bicarbonates such as sodium hydrogen carbonate, potassium hydrogen carbonate and the like, alkaline earth metal hydroxides such as magnesium hydroxide, calcium hydroxide and the like, alkaline earth metal carbonates such as magnesium carbonate, calcium carbonate and the like.

As the acid in the case of use of an acidic aqueous solution, for example, hydrogen chloride, hydrogen bromide, sulfuric acid, phosphoric acid and the like can be used.

The use amount of these water, base and acid is not particularly restricted. An amount necessary for dissolution of inorganic materials generated by hydrolysis of a reducing agent can be used, alternatively, an amount just necessary for hydrolysis of a reducing agent may be used, and inorganic components generated may be removed by filtration and the like.

Examples of the organic solvent separable from water described above include aliphatic hydrocarbon solvents such as hexane, heptane, cyclohexane and the like; aromatic solvents such as toluene, xylene, monochlorobenzene, dichlorobenzene and the like; ether solvents such as methyl tert-butyl ether, 1,2-dimethoxyethane and the like; halogenated hydrocarbon solvents such as dichloromethane, dichloroethane, chlorobutane and the like; ketone solvents such as methyl ethyl ketone, methyl isobutyl ketone and the like; ester solvents such as methyl acetate, ethyl acetate, butyl acetate and the like; etc.

The organic layer obtained by liquid-partitioning may be further subjected to washing with water, washing with basic water, washing with acidic water, and the like.

Thus obtained solution containing N-protected pyrrolidinols (3) may be used as it is in the subsequent step, or may be once isolated by solvent concentration and the like. Further, it may be purified by a method such as column chromatography, re-crystallization or the like.

R¹, R², R³, R⁴, R⁵ and R⁶ in N-protected pyrrolidinols (3) represent the same meanings as for R¹, R², R³, R⁴, R⁵ and R⁶ in N-protected lactams (2).

Specific examples of N-protected pyrrolidinols (3) include 3-tert-butoxycarbonyl-3-azabicyclo[3.1.0]hexan-2-ol, 3-tert-butoxycarbonyl-3-azabicyclo[3.2.0]heptan-2-ol, 3-tert-butoxycarbonyl-3-azabicyclo[3.3.0]octan-2-ol, 8-tert-butoxycarbonyl-8-azabicyclo[4.3.0]nonan-7-ol, 3-tert-butoxycarbonyl-6,6-dimethyl-3-azabicyclo[3.1.0]hexan-2-ol, 3-tert-butoxycarbonyl-6,6-dichloro-3-azabicyclo[3.1.0]hexan-2-ol, 3-tert-butoxycarbonyl-6,6-difluoro-3-azabicyclo[3.1.0]hexan-2-ol, 3-tert-butoxycarbonyl-1-phenyl-3-azabicyclo[3.1.0]hexan-2-ol, 4-tert-butoxycarbonyl-4-azatricyclo[5.2.1.0^{2,6}]deca-3-ol, 4-tert-butoxycarbonyl-4-azatricyclo[5.2.2.0^{2,6}]undeca-3-ol, 8-tert-butoxycarbonyl-8-azabicyclo[4.3.0]nonan-3-ene-7-ol, 4-tert-butoxycarbonyl-4-azatricyclo[5.2.1.0^{2,6}]deca-8-ene-3-ol, 4-tert-butoxycarbonyl-4-azatricyclo[5.2.2.0^{2,6}]undeca-8-ene-3-ol, 7-tert-butoxycarbonyl-3,3-dimethyl-2,4-dioxa-7-azabicyclo[3.3.0]octan-6-ol, 7-tert-butoxycarbonyl-3-phenyl-2,4-dioxa-7-azabicyclo[3.3.0]octan-6-ol, 7-tert-butoxycarbonyl-3,3-dimethyl-2-oxa-7-azabicyclo[3.3.0]octan-6-ol, 4-tert-butoxycarbonyl-4-aza-10-oxatricyclo[5.2.1.0^{2,6}]deca-3-ol, 4-tert-butoxycarbonyl-4-aza-10-oxa-tricyclo[5.2.1.0^{2,6}]deca-8-ene-3-ol, 2-tert-butoxycarbonyl-2-azabicyclo[3,1,0]hexan-3-ol, 2-tert-butoxycarbonyl-2-azabicyclo[3.3.0]octan-3-ol, 7-tert-butoxycarbonyl-7-azabicyclo[4.3.0]nonan-8-ol, 2-tert-butoxycarbonyl-2-azabicyclo[3.3.0]octa-7-ene-3-ol, 2-tert-butoxycarbonyl-2-azabicyclo[2.2.1]heptan-3-ol, and optically active bodies thereof, and the like. When optically active bodies are used as the N-protected lactams (2), the resultant N-protected pyrrolidinols (3) are usually optically active bodies.

Next, the step B of the present invention will be illustrated.

The step B is a step of reacting the N-protected pyrrolidinols (3) obtained in the step A with a cyanodizing agent in the presence of an acid catalyst, to obtain N-protected cyanopyrrolidines of the formula (4) [hereinafter, abbreviated as N-protected cyanopyrrolidines (4) in some cases.].

Examples of the cyanodizing agent include trimethylsilyl cyanide, hydrogen cyanide, sodium cyanide, potassium cyanide and the like. Preferably, trimethylsilyl cyanide is used.

The use amount of the above-described cyanodizing agent is usually in the range of 0.8 to 10-mole ratio, preferably 1 to 3-mole ratio with respect to N-protected pyrrolidinols (3).

Examples of the acid catalyst include boron trifluoride complexes such as a boron trifluoride-diethyl ether complex, boron trifluoride-dimethyl sulfide complex and the like, and zinc chloride, titanium (IV) chloride, tin (IV) chloride, aluminum chloride, trimethylsilyl trifluoromethanesulfonate, and the like. As the acid catalyst, boron trifluoride complexes are preferably used.

In the case of use of the acid catalyst, the use amount of the acid catalyst is usually in the range of 0.1 to 5-mole ratio, preferably 0.5 to 3-mole ratio with respect to N-protected pyrrolidinols (3).

The above-described cyanodizing reaction is usually carried out in an organic solvent. Examples of the organic solvent to be used in the cyanodizing reaction include aliphatic hydrocarbon solvents such as hexane, heptane, cyclohexane and the like; aromatic solvents such as toluene, xylene, monochlorobenzene, dichlorobenzene, trifluorotoluene and the like; ether solvents such as tetrahydrofuran, methyl tert-butyl ether, diethyl ether, 1,4-dioxane, 1,2-dimethoxyethane and the like; halogenated hydrocarbon solvents such as dichloromethane, dichloroethane, chlorobutane and the like; nitrile solvents such as acetonitrile, propionitrile and the like; ester solvents such as ethyl acetate, butyl acetate and the like; etc. These organic solvents may be used in admixture of two or more.

The use amount of the organic solvent is usually in the range of 1 to 100-weight ratio, preferably 2 to 20-weight ratio with respect to N-protected pyrrolidinols (3).

As the method for mixing the raw materials and reaction reagents described above in the cyanodizing reaction, mentioned are, for example, a method of dropping N-protected pyrrolidinols (3) and a cyanodizing agent into a solution containing an acid catalyst, a method of dropping an acid catalyst into a solution containing N-protected pyrrolidinols (3) and a cyanodizing agent, a method of dropping N-protected pyrrolidinols (3) into a solution containing an acid catalyst and a cyanodizing agent, a method of dropping a cyanodizing agent into a solution containing N-protected pyrrolidinols (3) and an acid catalyst; and other methods. As the preferable method in the cyanodizing reaction, a method of dropping N-protected pyrrolidinols (3) and a cyanodizing agent into a solution containing an acid catalyst is mentioned.

The reaction temperature in the above-described cyanodizing reaction is usually in the range of -100 to 10°C, preferably -80 to -10°C.

After completion of such a cyanodizing reaction, a solution containing N-protected cyanopyrrolidines (4) is obtained, and after completion of the reaction, a post-treatment operation is usually carried out for removal of an excess amount of cyanodizing agent and acid catalyst in the reaction liquid.

In the post-treatment operation, for example, a reaction solution and water are mixed, then, if necessary, an organic solvent separable from water is added to cause liquid-partitioning, thus, an excess amount of cyanodizing agent and acid catalyst, and inorganic components obtained by hydrolysis thereof, can be distributed in an aqueous layer. Meanwhile, the aimed compound, N-protected cyanopyrrolidines (4) can be distributed into an organic layer. Further, when pH in the post treatment is alkaline, a cyanodizing agent, acid catalyst and inorganic components obtained by hydrolysis thereof can be distributed efficiently into an aqueous layer, thus, it is preferable that, in the above-described post treatment, the reaction solution and water are mixed, then, a base is added to make it alkaline, or a basic aqueous solution is used as the water to be mixed with the reaction solution. The range of pH in the post-treatment operation is usually from 7 to 14, preferably 9 to 13.

As the base to be used in the post-treatment operation, inorganic bases such as alkali metal hydroxides such as sodium hydroxide, potassium hydroxide and the like, alkali metal carbonates such as sodium carbonate, potassium carbonate and the like, alkali metal bicarbonates such as sodium hydrogen carbonate, potassium hydrogen carbonate and the like; etc are used.

Examples of the above-described organic solvent separable from water include aliphatic hydrocarbon solvents such as hexane, heptane, cyclohexane and the like; aromatic solvents such as toluene, xylene, monochlorobenzene, dichlorobenzene and the like; ether solvents such as methyl tert-butyl ether, 1,2-dimethoxyethane and the like; halogenated hydrocarbon solvents such as dichloromethane, dichloroethane, chlorobutane and the like; ketone solvents such as methyl ethyl ketone, methyl isobutyl ketone and the like; ester solvents such as methyl acetate, ethyl acetate, butyl acetate and the like; etc.

The resultant organic layer may further be subjected to washing with water, washing with basic water, washing with acidic water, and the like.

For decomposing a toxic cyanodizing agent, it is preferable to carry out a post-treatment operation using an oxidizing agent. As the oxidizing agent, for example, sodium hypochlorite, hydrogen peroxide and the like are used. The treatment with an oxidizing agent is preferably carried out under a basic condition. In this case, the range of pH is usually from 7 to 14, preferably 9 to 13. In the above-described post treatment using a basic aqueous solution, an oxidizing agent may be added in performing the treatment.

After the above-described treatment using an oxidizing agent, neutralization may further be carried out using an acid, and additionally, a treatment with a reducing agent may be carried out for the purpose of decomposing an excess amount of oxidizing agent. Examples of the acid include aqueous solutions of inorganic acids such as hydrochloric acid, hydrobromic acid, sulfuric acid, phosphoric acid and the like, aqueous solutions of organic acids such as acetic acid, citric acid and the like; etc. Examples of the reducing agent include aqueous solutions of sodium sulfite, sodium thiosulfate and the like.

Thus obtained solution containing N-protected cyanopyrrolidines (4) may be used as it is in the subsequent step, or may be once isolated by solvent concentration and the like. Further, it may be purified by a method such as column chromatography, re-crystallization or the like.

R¹, R², R³, R⁴, R⁵ and R⁶ in N-protected cyanopyrrolidines (4) represent the same meanings as for R¹, R², R³, R⁴, R⁵ and R⁶ defined in N-protected pyrrolidinones (2).

Examples of the N-protected cyanopyrrolidines (4) include 3-tert-butoxycarbonyl-3-azabicyclo[3.1.0]hexane-2-carbonitrile, 3-tert-butoxycarbonyl-3-azabicyclo[3.2.0]heptane-2-carbonitrile, 3-tert-butoxycarbonyl-3-azabicyclo[3.3.0]octan-2-carbonitrile, 8-tert-butoxycarbonyl-8-azabicyclo[4.3.0]nonan-7-carbonitrile, 3-tert-butoxycarbonyl-6,6-dimethyl-3-azabicyclo[3.1.0]hexane-2-carbonitrile, 3-tert-butoxycarbonyl-6,6-dichloro-3-azabicyclo[3.1.0]hexane-2-carbonitrile, 3-tert-butoxycarbonyl-6,6-difluoro-3-azabicyclo[3.1.0]hexane-2-carbonitrile, 3-tert-butoxycarbonyl-1-phenyl-3-azabicyclo[3.1.0]hexane-2-carbonitrile, 4-tert-butoxycarbonyl-4-azatricyclo[5.2.1.0^{2,6}]deca-3-carbonitrile, 4-tert-butoxycarbonyl-4-azatricyclo[5.2.2.0^{2,6}]undeca-3-carbonitrile, 8-tert-butoxycarbonyl-8-azabicyclo[4.3.0]nonan-3-ene-7-carbonitrile, 4-tert-butoxycarbonyl-4-azatricyclo[5.2.1.0^{2,6}]deca-8-ene-3-carbonitrile, 4-tert-butoxycarbonyl-4-azatricyclo[5.2.2.0^{2,6}]undeca-8-ene-3-carbonitrile, 7-tert-butoxycarbonyl-3,3-dimethyl-2,4-dioxa-7-azabicyclo[3.3.0]octan-6-carbonitrile, 7-tert-butoxycarbonyl-3-phenyl-2,4-dioxa-7-azabicyclo[3.3.0]octan-6-carbonitrile, 7-tert-butoxycarbonyl-3,3-dimethyl-2-oxa-7-azabicyclo[3.3.0]octan-6-carbonitrile, 4-tert-butoxycarbonyl-4-aza-10-oxa-tricyclo[5.2.1.0^{2,6}]deca-3-carbonitrile, 4-tert-butoxycarbonyl-4-aza-10-oxatricyclo[5.2.1.0^{2,6}]deca-8-ene-3-carbonitrile, 2-tert-butoxycarbonyl-2-azabicyclo[3,1,0]hexane-3-carbonitrile, 2-tert-butoxycarbonyl-2-azabicyclo[3.3.0]octan-3-carbonitrile, 7-tert-butoxycarbonyl-7-azabicyclo[4.3.0]nonan-8-carbonitrile, 2-tert-butoxycarbonyl-2-azabicyclo[3.3.0]octa-7-ene-3-carbonitrile, 2-tert-butoxycarbonyl-2-azabicyclo[2.2.1]heptane-3-carbonitrile and the like.

As the N-protected cyanopyrrolidines (4), optically active bodies of the above-exemplified compounds, and the like are mentioned. When optically active bodies are used as the N-protected pyrrolidinols (3), the resultant N-protected cyanopyrrolidines (4) are usually optically active bodies.

Next, the step C of the present invention will be illustrated.

The step C is a step of reacting the N-protected cyanopyrrolidines (4) obtained in the step B with alcohols in the presence of a base, to obtain imidates of the formula (5) [hereinafter, abbreviated as imidates (5) in some cases.] as an intermediate, then, treating with an acid to obtain N-protected prolines of the formula (6) [hereinafter, abbreviated as N-protected prolines (6) in some cases.].

Examples of the alcohols include alkyl alcohols having 1 to 10 carbon atoms, alkenyl alcohols having 2 to 10 carbon atoms, aralkyl alcohols having 7 to 20 carbon atoms and the like such as methanol, ethanol, 1-propanol, 2-propanol, 1-butanol, 2-butanol, 2-methyl-1-propanol, 2-methyl-2-propanol, allyl alcohol, benzyl alcohol, p-methoxybenzyl alcohol, and the like.

The above-described alcohols can be used also as a reaction solvent. The use amount of the above-described alcohols is usually in the range of 0.2 to 50-weight ratio, preferably 2 to 20-weight ratio with respect to N-protected cyanopyrrolidines (4).

Examples of other reaction solvents than the above-described alcohols include aliphatic hydrocarbon solvents such as hexane, heptane, cyclohexane and the like; aromatic solvents such as toluene, xylene, monochlorobenzene, dichlorobenzene and the like; ether solvents such as tetrahydrofuran, methyl tert-butyl ether, 1,4-dioxane, 1,2-dimethoxyethane and the like; halogenated hydrocarbon solvents such as dichloromethane, dichloroethane, chlorobutane and the like; ketone solvents such as acetone, methyl ethyl ketone, methyl isobutyl ketone and the like; ester solvents such as methyl acetate, ethyl acetate, butyl acetate and the like; amide solvents such as N,N-dimethylformamide, N,N-dimethylacetamide, N-methylpyrrolidinone and the like; etc. These solvents may be used singly or in admixture of two or more.

Examples of the base include alkali metal hydroxides such as sodium hydroxide, potassium hydroxide and the like; alkali metal carbonate addition salts such as sodium carbonate, potassium carbonate, cesium carbonate and the like; alkali metal alcoholates such as sodium methoxide, sodium ethoxide and the like; alkali metal hydrides such as sodium hydride, potassium hydride and the like; organic bases such as triethylamine, diisopropylethylamine, 1,8-diazabicyclo[5.4.0]undeca-7-ene, pyridine and the like; etc.

The use amount of the above-described base is usually in the range of 0.5 to 10-mole ratio, preferably 1 to 5-mole ratio with respect to N-protected cyanopyrrolidines (4).

The reaction temperature in obtaining imidates (5) from N-protected cyanopyrrolidines (4) is usually in the range of -30 to 30°C, preferably -20 to 10°C.

A solution containing imidates (5) obtained in the above-described reaction can be derived into N-protected prolines (6) by an acid treatment such as mixing with acidic water, or the like.

The treatment with acidic water includes, for example, a method of adding acidic water into a reaction solution containing imidates (5), a method of adding the above-described reaction solution into acidic water, a method of mixing the above-described reaction solution and water, then, adding acidic water, and other methods.

In the above-described reaction, the reaction temperature is usually in the range of -20 to 20°C, preferably -10 to 10°C, so as to prevent side reactions such as decomposition of imidates (5), hydrolysis of an ester group in N-protected prolines (6), de-protection of an N-protective group in N-protected prolines (6), and the like. For suppressing the above-described side reactions and decomposition reactions, the above-described reaction solution and acidic water may be poured simultaneously so as to keep pH in dropping neutral.

Examples of the above-described acidic water include aqueous solutions of inorganic acids such as hydrochloric acid, hydrobromic acid, sulfuric acid, phosphoric acid and the like, aqueous solutions of organic acids such as acetic acid, citric acid and the like; etc.

The use amount of these acids is usually in the range of 1 to 20-mole ratio with respect to N-protected cyanopyrrolidines (4), and preferably is an amount obtained by adding 0.5 to 2-mole ratio with respect to N-protected cyanopyrrolidines (4) to the amount of an acid required for neutralizing a base used in the reaction of obtaining imidates (5).

If necessary, an organic solvent separable from water can be added to the N-protected prolines (6) in the reaction solution, thereby, distributing the N-protected prolines (6) into an organic layer.

Examples of the organic solvent separable from water to be used in distribution include aliphatic hydrocarbon solvents such as hexane, heptane, cyclohexane and the like; aromatic solvents such as toluene, xylene, monochlorobenzene, dichlorobenzene and the like; ether solvents such as methyl tert-butyl ether, 1,2-dimethoxyethane and the like; halogenated hydrocarbon solvents such as dichloromethane, dichloroethane, chlorobutane and the like; ketone solvents such as methyl ethyl ketone, methyl isobutyl ketone and the like; ester solvents such as methyl acetate, ethyl acetate, butyl acetate and the like; etc.

The use amount of the organic solvent is usually in the range of 1 to 100-weight ratio, preferably 2 to 20-weight ratio with respect to N-protected cyanopyrrolidines (4).

The resultant organic layer may further be subjected to washing with water, washing with basic water, washing with acidic water, and the like.

Thus obtained solution containing N-protected prolines (6) may be used as it is in the subsequent step, or may be once isolated by solvent concentration and the like. It may be further purified by a method such as column chromatography, re-crystallization or the like.

R¹, R², R³, R⁴, R⁵ and R⁶ in N-protected prolines (6) represent the same meanings as for R¹, R², R³, R⁴, R⁵ and R⁶ defined in N-protected pyrrolidinones (2).

R⁷ represents an optionally substituted alkyl group having 1 to 10 carbon atoms, optionally substituted alkenyl group having 2 to 10 carbon atoms or optionally substituted aralkyl group having 7 to 20 carbon atoms.

Specific examples of N-protected prolines (6) include compounds such as 3-tert-butoxycarbonyl-3-azabicyclo[3.1.0]hexane-2-carboxylic acid methyl ester, 3-tert-butoxycarbonyl-3-azabicyclo[3.2.0]heptane-2-carboxylic acid methyl ester, 3-tert-butoxycarbonyl-3-azabicyclo[3.3.0]octane-2-carboxylic acid methyl ester, 8-tert-butoxycarbonyl-8-azabicyclo[4.3.0]nonane-7-carboxylic acid methyl ester, 3-tert-butoxycarbonyl-6,6-dimethyl-3-azabicyclo[3.1.0]hexane-2-carboxylic acid methyl ester, 3-tert-butoxycarbonyl-6,6-dichloro-3-azabicyclo[3.1.0]hexane-2-carboxylic acid methyl ester, 3-tert-butoxycarbonyl-6,6-difluoro-3-azabicyclo[3.1.0]hexane-2-carboxylic acid methyl ester, 3-tert-butoxycarbonyl-1-phenyl-3-azabicyclo[3.1.0]hexane-2-carboxylic acid methyl ester, 4-tert-butoxycarbonyl-4-azatricyclo[5.2.1.0^{2,6}]deca-3-carboxylic acid methyl ester, 4-tert-butoxycarbonyl-4-azatricyclo[5.2.2.0^{2,6}]undeca-3-carboxylic acid methyl ester, 8-tert-butoxycarbonyl-8-azabicyclo[4.3.0]nonan-3-ene-7-carboxylic acid methyl ester, 4-tert-butoxycarbonyl-4-azatricyclo[5.2.1.0^{2,6}]deca-8-ene-3-carboxylic acid methyl ester, 4-tert-butoxycarbonyl-4-azatricyclo[5.2.2.0^{2,6}]undeca-8-ene-3-carboxylic acid methyl ester, 7-tert-butoxycarbonyl-3,3-dimethyl-2,4-dioxa-7-azabicyclo[3.3.0]octan-6-carboxylic acid methyl ester, 7-tert-butoxycarbonyl-3-phenyl-2,4-dioxa-7-azabicyclo[3.3.0]octan-6-carboxylic acid methyl ester, 7-tert-butoxycarbonyl-3,3-dimethyl-2-oxa-7-azabicyclo[3.3.0]octan-6-carboxylic acid methyl ester, 4-tert-butoxycarbonyl-4-aza-10-oxa-tricyclo[5.2.1.0^{2,6}]deca-3-carboxylic acid methyl ester, 4-tert-butoxycarbonyl-4-aza-10-oxa-tricyclo[5.2.1.0^{2,6}]deca-8-ene-3-carboxylic acid methyl ester, 2-tert-butoxycarbonyl-2-azabicyclo[3,1,0]hexane-3-carboxylic acid methyl ester, 2-tert-butoxycarbonyl-2-azabicyclo[3.3.0]octan-3-carboxylic acid methyl ester, 7-tert-butoxycarbonyl-7-azabicyclo[4.3.0]nonan-8-carboxylic acid methyl ester, 2-tert-butoxycarbonyl-2-azabicyclo[3.3.0]octa-7-ene-3-carboxylic acid methyl ester, 2-tert-butoxycarbonyl-2-azabicyclo[2.2.1]heptane-3-carboxylic acid methyl ester, and the like.

Also mentioned are compounds obtained by substitution of a methyl ester group in these compounds with an ethyl ester group, n-propyl ester group, isopropyl ester group, n-butyl ester group, sec-butyl ester group, isobutyl ester group, tert-butyl ester group, allyl ester group, benzyl ester group, p-methoxybenzyl ester group, and the like.

Further, optically active bodies of the above-described compounds are also mentioned. When optically active bodies are used as the N-protected cyanopyrrolidines (4), the resultant N-protected prolines (6) are usually optically active bodies.

Next, the step D of the present invention will be illustrated.

The step D is a step of treating the N-protected prolines (6) obtained in the step C with an acid to deprotect an N-protective group in the N-protected prolines (6), thereby obtaining a proline derivative of the formula (1) [hereinafter, abbreviated as proline derivative (1) in some cases.].

Examples of the de-protection reaction method include a method of dropping an acid or a solution containing an acid into a solution prepared by dissolving N-protected prolines (6) in a solvent, a method of blowing an acid in the form of gas into a solution prepared by dissolving N-protected prolines (6) in a solvent, a method of dropping a solution containing N-protected prolines (6) into an acid or a solution containing an acid, and other methods.

Examples of the above-described acid include inorganic acids such as hydrogen chloride, hydrogen bromide, sulfuric acid and the like, and organic acids such as methanesulfonic acid, trifluoroacetic acid and the like.

The use amount of the above-described acid is usually in the range of 0.5 to 20-mole ratio, preferably 1 to 10-mole ratio with respect to N-protected prolines (6).

The de-protection reaction is usually carried out in a solvent. Example of the above-described solvent include alcohol solvents such as methanol, ethanol, 1-propanol, 2-propanol, 1-butanol, 2-butanol, 2-methyl-1-propanol, 2-methyl-2-propanol and the like; aliphatic hydrocarbon solvents such as hexane, heptane, cyclohexane and the like; aromatic solvents such as toluene, xylene, monochlorobenzene, dichlorobenzene and the like; ether solvents such as tetrahydrofuran, methyl tert-butyl ether, 1,4-dioxane, 1,2-dimethoxyethane and the like; halogenated hydrocarbon solvents such as dichloromethane, dichloroethane, chlorobutane and the like; ester solvents such as methyl acetate, ethyl acetate, butyl acetate and the like; and water, and the like. These solvents may be used singly or in admixture of two or more.

When alcohol solvents and ester solvents are used, it is preferable to use an alcohol solvent or ester solvent corresponding to an ester group R⁷ in N-protected prolines (6), so as not to cause a transesterification reaction with the ester group in N-protected prolines (6).

For example, in the case of production of a compound in which R⁷ in N-protected prolines (6) is a methyl group, it is preferable to use methanol as the corresponding alcohol solvent. In the case of production of a compound in which R⁷ is an ethyl group, it is preferable to use ethyl acetate as the corresponding ester solvent.

The use amount of the above-described organic solvent is usually in the range of 0.5 to 100-weight ratio, preferably 1 to 20-weight ratio with respect to N-protected prolines (6).

The reaction temperature in the de-protection reaction is usually in the range of -20 to 100°C, preferably 0 to 50°C.

Thus, a proline derivative (1) is obtained, and after completion of the above-described de-protection reaction, the proline derivative (1) is usually present as an addition salt of an acid used in the reaction, and by concentrating the reaction solution to dryness, an acid addition salt of the proline derivative (1) can be taken out.

When the acid addition salt of the proline derivative (1) deposits as solid after completion of the reaction, the aimed acid addition salt of the proline derivative (1) can be isolated by filtration and the like. Further, when the deposition amount of the acid addition salt of the proline derivative (1) is small, its deposition amount can be increased by adding a poor solvent for the acid addition salt of the proline derivative (1). Also in the case of no deposition of the acid addition salt of the proline derivative (1), the acid addition salt of the proline derivative (1) can be crystallized and taken out as solid by adding a poor solvent.

By cooling the reaction solution after completion of the de-protection reaction, the acid addition salt of the proline derivative (1) may be crystallized, and the deposition amount of the acid addition salt of the proline derivative (1) can be increased.

Examples of the above-described poor solvent include organic solvents such as aliphatic hydrocarbon solvents such as hexane, heptane, cyclohexane and the like; aromatic solvents such as toluene, xylene, monochlorobenzene, dichlorobenzene and the like; ether solvents such as tetrahydrofuran, methyl tert-butyl ether, 1,4-dioxane, 1,2-dimethoxyethane and the like; halogenated hydrocarbon solvents such as dichloromethane, dichloroethane, chlorobutane and the like; ester solvents such as methyl acetate, ethyl acetate, butyl acetate and the like; etc. These organic solvents may be used singly or in admixture of two or more.

The use amount of the above-described organic solvent is usually in the range of 0 to 200-weight ratio, preferably 1 to 50-weight ratio with respect to N-protected prolines (6).

The resultant acid addition salt of the proline derivative (1) may be further purified by a method such as re-crystallization and the like.

For example, by mixing solid of the acid addition salt of the proline derivative (1) obtained in the above-described operation with water and an organic solvent separable from water, and neutralizing an acid forming the addition salt using a base, a free proline derivative (1) can be extracted into the organic solvent. Further, also by adding a base for neutralizing the acid used and water, and if necessary, an organic solvent separable from water, to the reaction solution obtained after the de-protection reaction, and performing extraction, a free proline derivative (1) can be obtained in the organic solvent.

The extraction operation with the organic solvent may be repeated. Extraction efficiency can also be enhanced by adding an inorganic salt and the like.

Examples of the organic solvent separable from water include aliphatic hydrocarbon solvents such as hexane, heptane, cyclohexane and the like; aromatic solvents such as toluene, xylene, monochlorobenzene, dichlorobenzene and the like; ether solvents such as methyl tert-butyl ether, 1,2-dimethoxyethane and the like; halogenated hydrocarbon solvents such as dichloromethane, dichloroethane, chlorobutane and the like; ketone solvents such as methyl ethyl ketone, methyl isobutyl ketone and the like; ester solvents such as methyl acetate, ethyl acetate, butyl acetate and the like; etc.

Examples of the base include inorganic bases such as alkali metal hydroxides such as sodium hydroxide, potassium hydroxide and the like; alkali metal carbonates such as sodium carbonate, potassium carbonate and the like; alkali metal bicarbonates such as sodium hydrogen carbonate, potassium hydrogen carbonate and the like; alkali metal phosphates such as trisodium phosphate, tripotassium phosphate, disodium hydrogen phosphate, dipotassium hydrogen phosphate and the like; organic bases such triethylamine, pyridine, piperidine and the like; and ammonia and the like.

The inorganic salt to be added for enhancing the extraction efficiency is not particularly restricted, and for example, sodium chloride, potassium chloride, ammonium chloride, sodium hydrogen carbonate, potassium hydrogen carbonate and the like are mentioned.

The resultant organic layer contains a free proline derivative (1), and the aimed free proline derivative (1) can be isolated by a method such as concentration of the organic solvent, and the like. The free proline derivative (1) gives rise to a dehydration reaction, thereby forming for example a dimer such as diketopiperazine and the like in some cases, thus, the concentration of the organic solvent is preferably carried out at a temperature which is as low as possible, usually about -10°C to 40°C. The free proline derivative (1) obtained by concentration of the organic solvent and the like may be purified by a method such as column chromatography, re-crystallization and the like.

The production method of the present invention is also a method containing three steps: the step A and step B described above, and a step E described later.

The step E of the present invention will be illustrated.

The step E is a step of reacting N-protected cyanopyrrolidines of the formula (4) obtained via the step A and the step B with alcohols and an acid, to produce a proline derivative of the formula (1) or acid addition salt thereof.

The step E can be carried out in the same manner as in the step D excepting that N-protected cyanopyrrolidines (4) are used instead of N-protected prolines (6) as a raw material compound in the step D. As the alcohols and acid to be used, the same compounds as described above are mentioned.

R¹, R², R³, R⁴, R⁵ and R⁶ in the proline derivative (1) represent the same meanings as for R¹, R², R³, R⁴, R⁵ and R⁶ defined in the N-protected pyrrolidinones (2).

R⁷ represents the same meaning as for R⁷ defined in the N-protected prolines (6).

Examples of the proline derivative (1) include 3-azabicyclo[3.1.0]hexane-2-carboxylic acid methyl ester, 3-azabicyclo[3.2.0]heptane-2-carboxylic acid methyl ester, 3-azabicyclo[3.3.0]octane-2-carboxylic acid methyl ester, 8-azabicyclo[4.3.0]nonane-7-carboxylic acid methyl ester, 6,6-dimethyl-3-azabicyclo[3.1.0]hexane-2-carboxylic acid methyl ester, 6,6-dichloro-3-azabicyclo[3.1.0]hexane-2-carboxylic acid methyl ester, 6,6-difluoro-3-azabicyclo[3.1.0]hexane-2-carboxylic acid methyl ester, 1-phenyl-3-azabicyclo[3.1.0]hexane-2-carboxylic acid methyl ester, 4-azatricyclo[5.2.1.0^{2,6}]deca-3-carboxylic acid methyl ester, 4-azatricyclo[5.2.2.0^{2,6}]undeca-3-carboxylic acid methyl ester, 8-azabicyclo[4.3.0]nonan-3-ene-7-carboxylic acid methyl ester, 4-azatricyclo[5.2.1.0^{2,6}]deca-8-ene-3-carboxylic acid methyl ester, 4-azatricyclo[5.2.2.0^{2,6}]undeca-8-ene-3-carboxylic acid methyl ester, 3,3-dimethyl-2,4-dioxa-7-azabicyclo[3.3.0]octan-6-carboxylic acid methyl ester, 3-phenyl-2,4-dioxa-7-azabicyclo[3.3.0]octan-6-carboxylic acid methyl ester, 3,3-dimethyl-2-oxa-7-azabicyclo[3.3.0]octan-6-carboxylic acid methyl ester, 4-aza-10-oxatricyclo[5.2.1.0^{2,6}]deca-3-carboxylic acid methyl ester, 4-aza-10-oxa-tricyclo[5.2.1.0^{2,6}]deca-8-ene-3-carboxylic acid methyl ester, 2-azabicyclo[3,1,0]hexane-3-carboxylic acid methyl ester, 2-azabicyclo[3.3.0]octan-3-carboxylic acid methyl ester, 7-azabicyclo[4.3.0]nonan-8-carboxylic acid methyl ester, 2-azabicyclo[3.3.0]octa-7-ene-3-carboxylic acid methyl ester, 2-azabicyclo[2.2.1]heptane-3-carboxylic acid methyl ester, and the like.

Further, compounds obtained by substituting the above-described methyl ester by an ethyl ester, n-propyl ester, isopropyl ester, n-butyl ester, sec-butyl ester, isobutyl ester, tert-butyl ester, allyl ester, benzyl ester or p-methoxybenzyl ester, and the like, are mentioned.

Furthermore, acid addition salts obtained by addition of an inorganic acid such as hydrogen chloride, hydrogen bromide, sulfuric acid and the like, and acid addition salts obtained by addition of an organic acid such as methanesulfonic acid, trifluoroacetic acid and the like, to the above-described proline derivative (1), are also exemplified.

Still further, optically active bodies of the above-described proline derivatives (1) or acid addition salts thereof are also mentioned. When optically active bodies are used as the N-protected prolines (6), the resultant proline derivatives (1) or acid addition salts thereof are usually optically active bodies.

According to the present invention, a proline derivative (1) or acid addition salt thereof can be produced simply and industrially advantageously from the correspondent pyrrolidinones.

The proline derivative (1) or acid addition salt thereof obtained in the present invention is useful as a chemical raw material or medical-agricultural drug intermediate, and for example, can be used suitably as a production intermediate for the following compound (see, WO 2004/113295) as one of anti-hepatitis C drugs (HCV drug).

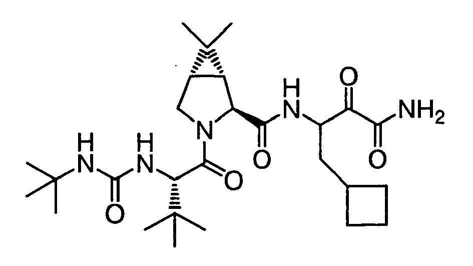

### EXAMPLES

The present invention will be illustrated further in detail based on examples below, but it is needless to say that the present invention is not limited to these examples.

### Example 1

Synthesis Example of (1R,5S)-3-tert-butoxycarbonyl-6,6-dimethyl-3-azabicyclo[3.1.0]hexan-2-ol 25.0 g (111 mmol) of (1R,5S)-3-tert-butoxycarbonyl-6,6-dimethyl-3-azabicyclo[3.1.0]hexan-2-one and 25 g of tetrahydrofuran were mixed, and cooled down to -10°C, then, 105 g of a toluene solution (1 mol/l) of diisobutylaluminum hydride was dropped at -10°C over a period of 3 hours. After completion of dropping, the mixture was stirred for 1 hour at the same temperature, then, the reaction liquid was heated up to 20°C. After rising of temperature, 22 g of a 2% sodium hydroxide aqueous solution was dropped, then, the mixture was stirred at 25°C. The deposited crystal was filtrated, thereby obtaining a filtrate.

Next, the resultant materials on the filter were washed with 80 g of tetrahydrofuran, obtaining washing liquid.

The above-described filtrate and washing liquid were mixed to obtain a solution which was then washed three times with 22 g of a 2% sodium hydroxide aqueous solution. This washing includes mixing under stirring and liquid-partitioning under still standing.

The resultant organic layer was washed twice with 22 g of 15% saline. The organic layer obtained after washing was concentrated under reduced pressure condition, then, to the resultant concentration residue was added 75 g of toluene and 25 g of water and mixed before liquid-partitioning.

The resultant organic layer was concentrated under reduced pressure to distill off the solvent, obtaining 25.0 g of a solution containing 24.1 g (106 mmol) of (1R,5S)-3-tert-butoxycarbonyl-6,6-dimethyl-3-azabicyclo[3.1.0]hexan-2-ol.

The yield with respect to (1R,5S)-3-tert-butoxycarbonyl-6,6-dimethyl-3-azabicyclo[3.1.0]hexan-2-one was 95.5%.

The quantitative determination of 3-tert-butoxycarbonyl-6,6-dimethyl-3-azabicyclo[3.1.0]hexan-2-ol was performed by high performance liquid chromatography. As the column, ZORBAX SB-Phenyl, 4.6 mm φ x 250 mm, 5 pm, manufactured by Agilent was used.

The measurement results of ¹H-NMR (DMSO-d6) of (1R,5S)-3-tert-butoxycarbonyl-6,6-dimethyl-3-azabicyclo[3.1.0]hexan-2-ol are shown below.

δ = 5.22 to 4.99 m (1H), 3.43 dd (1H), 3.24 dd (1H), 1.38 s (9H), 1.47 to 1.13 m (2H), 0.96 s (3H), 0.82 s (3H)

### Example 2

### Synthesis Example of 3-tert-butoxycarbonyl-6,6-dimethyl-3-azabicyclo[3.1.0]hexan-2-ol

11.0 g (49 mmol) of 3-tert-butoxycarbonyl-6,6-dimethyl-3-azabicyclo[3.1.0]hexan-2-one and 196 g of tetrahydrofuran were mixed, and cooled down to -20°C, then, 59 ml of a tetrahydrofuran solution (1 mol/l) of lithium triethylborohydride was dropped at -20°C. After completion of dropping, the mixture was stirred for 2 hours at the same temperature, then, 142 g of a 7% sodium hydrogen carbonate aqueous solution was added, and heated up to 0°C. After rising of temperature, 13 g of 30% hydrogen peroxide water was dropped. After stirring at 0°C for 30 minutes, the solvent was distilled off by concentration under reduced pressure. To the residue obtained after solvent distilling off was added 100 ml of water and 100 ml of methyl tert-butyl ether and mixed before liquid partitioning. Further, the aqueous layer obtained after liquid-partitioning was subjected to an extraction and liquid-partitioning operation twice using 100 ml of methyl tert-butyl ether. The resultant organic layers were combined to give a solution to which sodium sulfate was added, and mixed, then, solid was removed by filtration, and the filtrate was concentrated under reduced pressure to distill off the solvent, obtaining 11.0 g of 3-tert-butoxycarbonyl-6,6-dimethyl-3-azabicyclo[3.1.0]hexan-2-ol in the form of white solid. The yield of 3-tert-butoxycarbonyl-6,6-dimethyl-3-azabicyclo[3.1.0]hexan-2-ol with respect to 3-tert-butoxycarbonyl-6,6-dimethyl-3-azabicyclo[3.1.0]hexan-2-one was 98.5%.

The measurement results by ¹H-NMR (DMSO-d6) of 3-tert-butoxycarbonyl-6,6-dimethyl-3-azabicyclo[3.1.0]hexan-2-ol are shown below.

δ = 5.48 to 5.45 m (1H), 3.42 dd (1H), 3.24 d (1H), 1.49 to 1.44 m (1H), 1.40 s (9H), 1.31 to 1.26 m (1H), 1.17 s (3H), 0.98 s (3H)

### Example 3

### Synthesis Example of 3-tert-butoxycarbonyl-6,6-dimethyl-3-azabicyclo[3.1.0]hexan-2-ol

0.50 g (2.2 mmol) of 3-tert-butoxycarbonyl-6,6-dimethyl-3-azabicyclo[3.1.0]hexan-2-one and 12.5 ml of tetrahydrofuran were mixed, and cooled down to -78°C, then, 3.3 ml of a toluene solution (1 mol/l) of diisobutylaluminum hydride was dropped over a period of 1 hour so as to keep the temperature at -70°C or lower.

After completion of dropping, the mixture was stirred at the same temperature for 1.5 hours, then, 8.7 ml of water was added and heated up to 25°C. Further, 5 ml of water and 10 ml of methyl tert-butyl ether were added, then, the deposited solid was removed by filtration. The resultant filtrate was liquid-partitioned, and an aqueous layer was again extracted and liquid-partitioned using 10 ml of methyl tert-butyl ether. All of the resultant organic layers were combined and mixed, and magnesium sulfate was added and mixed, then, solid was removed by filtration. The resultant filtrate was concentrated under reduced pressure to distill off the solvent, obtaining 0.48 g of 3-tert-butoxycarbonyl-6,6-dimethyl-3-azabicyclo[3.1.0]hexan-2-ol in the form of white solid. The yield with respect to 3-tert-butoxycarbonyl-6,6-dimethyl-3-azabicyclo[3.1.0]hexan-2-one was 94.4%.

### Example 4

### Synthesis Example of 3-tert-butoxycarbonyl-6,6-dimethyl-3-azabicyclo[3.1.0]hexan-2-ol

0.20 g (0.89 mmol) of 3-tert-butoxycarbonyl-6,6-dimethyl-3-azabicyclo[3.1.0]hexan-2-one and 10 ml of methanol were mixed and cooled down to -10°C, then, a solution composed of 0.072 g (3.3 mmol) of lithium borohydride and 15 ml of tetrahydrofuran was divided into two portions which were dropped separately at -10°C. The mixture was stirred for 17 hours at the same temperature, then, 2 g of water was added, and the mixture was heated up to 25°C. Further, 15 ml of water and 30 ml of methyl tert-butyl ether were added and mixed, causing liquid-partitioning. To the resultant organic layer was added magnesium sulfate and mixed, then, solid was removed by filtration, to obtain a solution containing 0.16 g of 3-tert-butoxycarbonyl-6,6-dimethyl-3-azabicyclo[3.1.0]hexan-2-ol with a yield with respect to 3-tert-butoxycarbonyl-6,6-dimethyl-3-azabicyclo[3.1.0]hexan-2-one of 80.1%.

### Example 5

### Synthesis Example of 3-tert-butoxycarbonyl-6,6-dimethyl-3-azabicyclo[3.1.0]hexan-2-ol

A solution composed of 0.50 g (2.2 mmol) of 3-tert-butoxycarbonyl-6,6-dimethyl-3-azabicyclo[3.1.0]hexan-2-one and 5 ml of tetrahydrofuran was dropped into 2.2 ml of a tetrahydrofuran solution (1 mol/l) of lithium aluminum hydride at -10°C over a period of 1 hour, and the mixture was stirred for 1.5 hours at -10°C. To this solution was added 0.13 g of water, and further, 0.32 g of a 10% sodium hydroxide aqueous solution, then, the mixture was heated up to 25°C. Further, 5 ml of water and 10 ml of methyl tert-butyl ether were added, then, the deposited solid was removed by filtration. The resultant filtrate was liquid-partitioned, and to an organic layer was added magnesium sulfate and mixed, then, solid was removed by filtration, to obtain a solution containing 0.38 g of 3-tert-butoxycarbonyl-6,6-dimethyl-3-azabicyclo[3.1.0]hexan-2-ol with a yield with respect to 3-tert-butoxycarbonyl-6,6-dimethyl-3-azabicyclo[3.1.0]hexan-2-one of 77.5%.

### Example 6

### Synthesis Example of (1R,2S,5S)-3-tert-butoxycarbonyl-6,6-dimethyl-3-azabicyclo[3.1.0]hexane-2-carbonitrile

1.97 g of a boron trifluoride-diethyl ether complex and 6.2 ml of toluene were mixed and cooled down to -60°C. Into this mixture, a solution prepared by mixing 5.8 g of a toluene solution containing 1.45 g (6.4 mmol) of (1R,5S)-3-tert-butoxycarbonyl-6,6-dimethyl-3-azabicyclo[3.1.0]hexan-2-ol obtained by the same manner as in Example 1, 1.37 g of trimethylsilyl cyanide and 2.5 ml of toluene was dropped over a period of 3 hours at -60 to -55°C. After completion of dropping, the mixture was stirred at -60 to -55°C, then, 35 g of an 8% sodium hydrogen carbonate aqueous solution was added, the mixture was heated up to 25°C, then, liquid-partitioned. The aqueous layer was again subjected to an extraction and liquid-partitioning operation using 10 ml of toluene. The resultant organic layers were combined and mixed, to this was added magnesium sulfate and mixed, then, solid was removed by filtration. The resultant solution was partially concentrated under reduced pressure to distill off the solvent, obtaining 6.68 g of a solution containing 1.35 g (5.7 mmol) of (1R,2S,5S)-3-tert-butoxycarbonyl-6,6-dimethyl-3-azabicyclo[3.1.0]hexane-2-carbonitrile.

The yield with respect to (1R,5S)-3-tert-butoxycarbonyl-6,6-dimethyl-3-azabicyclo[3.1.0]hexan-2-ol was 89.3%.

Quantitative determination of 3-tert-butoxycarbonyl-6,6-dimethyl-3-azabicyclo[3.1.0]hexane-2-carbonitrile was performed by gas chromatography [column was DB-1, 0.25 mmφ x 30 m, 0.25 µm manufactured by J&J].

The melting point of (1R,2S,5S)-3-tert-butoxycarbonyl-6,6-dimethyl-3-azabicyclo[3.1.0]hexane-2-carbonitrile was 45 to 46°C. The ¹H-NMR (DMSO-d6) data of (1R,2S,5S)-3-tert-butoxycarbonyl-6,6-dimethyl-3-azabicyclo[3.1.0]hexane-2-carbonitrile are shown below.

δ = 5.22 to 4.99 m (1H), 3.43 dd (1H), 3.24 dd (1H), 1.47 to 1.13 m (2H), 1.38 s (9H), 0.96 s (3H), 0.82 s (3H)

### Example 7

### Synthesis Example of (1R,2S,5S)-3-tert-butoxycarbonyl-6,6-dimethyl-3-azabicyclo[3.1.0]hexane-2-carbonitrile

7.01 g of a solution containing 1.34 g (5.7 mmol) of (1R,2S,5S)-3-tert-butoxycarbonyl-6,6-dimethyl-3-azabicyclo[3.1.0]hexane-2-carbonitrile was obtained in the same manner as in Example 6 excepting that a boron trifluoride-diethyl ether complex and toluene were mixed and cooled down to -40°C, and into this mixed liquid, a toluene solution containing (1R,5S)-3-tert-butoxycarbonyl-6,6-dimethyl-3-azabicyclo[3.1.0]hexan-2-ol and trimethylsilyl cyanide was dropped at -45 to -40°C over a period of 7 hours.

The yield with respect to (1R,5S)-3-tert-butoxycarbonyl-6,6-dimethyl-3-azabicyclo[3.1.0]hexan-2-ol was 88.6%.

### Example 8

### Synthesis Example of (1R,2S,5S)-3-tert-butoxycarbonyl-6,6-dimethyl-3-azabicyclo[3.1.0]hexane-2-carbonitrile

6.66 g of a solution containing 1.12 g (4.7 mmol) of (1R,2S,5S)-3-tert-butoxycarbonyl-6,6-dimethyl-3-azabicyclo[3.1.0]hexane-2-carbonitrile was obtained with a yield with respect to (1R,5S)-3-tert-butoxycarbonyl-6,6-dimethyl-3-azabicyclo[3.1.0]hexan-2-ol of 73.9% in the same manner as in Example 6 excepting that a boron trifluoride-diethyl ether complex and toluene were mixed and cooled down to -25°C, and a toluene solution containing (1R,5S)-3-tert-butoxycarbonyl-6,6-dimethyl-3-azabicyclo[3.1.0]hexan-2-ol and trimethylsilyl cyanide was dropped at -25 to -20°C over a period of 3 hours.

### Example 9

### Synthesis Example of (1R,2S,5S)-3-tert-butoxycarbonyl-6,6-dimethyl-3-azabicyclo[3.1.0]hexane-2-carbonitrile

0.81 g of a boron trifluoride-diethyl ether complex and 11.6 ml of methyl tert-butyl ether were mixed and cooled down to -40°C. Into this solution, a solution prepared by mixing 2.40 g of a toluene solution containing 0.60 g (2.6 mmol) of (1R,5S)-3-tert-butoxycarbonyl-6,6-dimethyl-3-azabicyclo[3.1.0]hexan-2-ol obtained by the same manner as in Example 1, and 0.53 g of trimethylsilyl cyanide and 4.6 ml of methyl tert-butyl ether was dropped at -45 to -35°C over a period of 3 hours. After completion of dropping, the mixture was stirred for 4 hours at -45 to -35°C, then, 16 g of an 8% sodium hydrogen carbonate aqueous solution was added and the mixture was heated up to 25°C, then, liquid-partitioned. The aqueous layer obtained by liquid-partitioning was again subjected to an extraction and liquid-partitioning operation using 10 ml of methyl tert-butyl ether. The resultant organic layers were combined, and to this was added magnesium sulfate and mixed, then, solid was removed by filtration. The resultant solution was concentrated under reduced pressure to distill off a part of the solvent, obtaining 6.06 g of a solution containing 0.49 g (2.1 mmol) of (1R,2S,5S)-3-tert-butoxycarbonyl-6,6-dimethyl-3-azabicyclo[3.1.0]hexane-2-carbonitrile with a yield with respect to (1R,5S)-3-tert-butoxycarbonyl-6,6-dimethyl-3-azabicyclo[3.1.0]hexan-2-ol of 79.2%.

### Example 10

### Synthesis Example of (1R,2S,5S)-3-tert-butoxycarbonyl-6,6-dimethyl-3-azabicyclo[3.1.0]hexane-2-carbonitrile

6.04 g of a solution containing 0.47 g (2.0 mmol) of (1R,2S,5S)-3-tert-butoxycarbonyl-6,6-dimethyl-3-azabicyclo[3.1.0]hexane-2-carbonitrile was obtained with a yield with respect to (1R,5S)-3-tert-butoxycarbonyl-6,6-dimethyl-3-azabicyclo[3.1.0]hexan-2-ol of 77.7% in the same manner as in Example 9 excepting that methyl tert-butyl ether used in the reaction was changed to 1-chlorobutane.

### Example 11

### Synthesis Example of (1R,2S,5S)-3-tert-butoxycarbonyl-6,6-dimethyl-3-azabicyclo[3.1.0]hexane-2-carbonitrile

6.00 g of a solution containing 0.38 g (1.6 mmol) of (1R,2S,5S)-3-tert-butoxycarbonyl-6,6-dimethyl-3-azabicyclo[3.1.0]hexane-2-carbonitrile was obtained in the same manner as in Example 9 excepting that methyl tert-butyl ether used in the reaction was changed to n-heptane. The yield with respect to (1R,5S)-3-tert-butoxycarbonyl-6,6-dimethyl-3-azabicyclo[3.1.0]hexan-2-ol was 61.5%.

### Example 12

### Synthesis Example of 3-tert-butoxycarbonyl-6,6-dimethyl-3-azabicyclo[3.1.0]hexane-2-carbonitrile

A solution prepared by mixing 0.20 g (0.88 mmol) of 3-tert-butoxycarbonyl-6,6-dimethyl-3-azabicyclo[3.1.0]hexan-2-ol obtained in the same manner as in Example 3, and 0.19 g of trimethylsilyl cyanide and 14 ml of methyl tert-butyl ether was cooled down to -78°C, and 0.27 g of a boron trifluoride-diethyl ether complex was dropped at -78 to - 68°C. The mixture was thermally insulated at the same temperature for 3 hours, then, heated up to -40°C and thermally insulated at the same temperature for 4 hours. To this was added 5.3 g of a 7% sodium hydrogen carbonate aqueous solution, and the mixture was heated up to 25°C before liquid-partitioning. The aqueous layer was further subjected to an extraction and liquid-partitioning operation twice using 10 ml of methyl tert-butyl ether. The resultant organic layers were all mixed, and to this was added sodium sulfate and mixed, then, solid was removed by filtration. The resultant solution was partially concentrated under reduced pressure to distill off the solvent, obtaining 0.20 g of a solution containing 0.17 g (0.73 mmol) of 3-tert-butoxycarbonyl-6,6-dimethyl-3-azabicyclo[3.1.0]hexane-2-carbonitrile.

The yield with respect to 3-tert-butoxycarbonyl-6,6-dimethyl-3-azabicyclo[3.1.0]hexan-2-ol was 83.2%.

The results of ¹H-NMR (CDCl₃) of 3-tert-butoxycarbonyl-6,6-dimethyl-3-azabicyclo[3.1.0]hexane-2-carbonitrile are shown below.

δ = 4.41 to 4.30 d (1H), 3.63 to 3.54 m (1H), 3.49 to 3.36 m (1H), 1.76 to 1.42 m (2H), 1.49 s (9H), 1.10 s (3H), 0.91 s (3H)

### Example 13

### Synthesis Example of 3-tert-butoxycarbonyl-6,6-dimethyl-3-azabicyclo[3.1.0]hexane-2-carbonitrile

0.27 g of a boron trifluoride-diethyl ether complex and 10 ml of methyl tert-butyl ether were mixed and adjusted to -40°C. Into this mixed liquid was added 0.18 g of trimethylsilyl cyanide, then, a solution prepared by mixing 0.77 g of a toluene solution containing 0.20 g (0.88 mmol) of 3-tert-butoxycarbonyl-6,6-dimethyl-3-azabicyclo[3.1.0]hexan-2-ol obtained in the same manner as in Example 3, and 4 ml of methyl tert-butyl ether was dropped at -45 to -35°C. After thermal insulation at the same temperature for 3 hours, 5.3 g of a 7% sodium hydrogen carbonate aqueous solution was added and the mixture was heated up to 25°C before liquid-partitioning. The aqueous layer was again subjected to an extraction and liquid-partitioning operation using 20 ml of methyl tert-butyl ether. The resultant organic layers were all mixed, then, magnesium sulfate was added and mixed, then, solid was removed by filtration. The resultant solution was partially concentrated under reduced pressure to distill off the solvent, obtaining 2.03 g of a solution containing 0.14 g (0.64 mmol) of 3-tert-butoxycarbonyl-6,6-dimethyl-3-azabicyclo[3.1.0]hexane-2-carbonitrile.

The yield with respect to 3-tert-butoxycarbonyl-6,6-dimethyl-3-azabicyclo[3.1.0]hexan-2-ol was 72.3%.

### Example 14

### Synthesis Example of 3-tert-butoxycarbonyl-6,6-dimethyl-3-azabicyclo[3.1.0]hexane-2-carbonitrile

A solution prepared by mixing 0.20 g (0.88 mmol) of 3-tert-butoxycarbonyl-6,6-dimethyl-3-azabicyclo[3.1.0]hexan-2-ol obtained in the same manner as in Example 3, and 12 ml of methyl tert-butyl ether was cooled down to -78°C.

Into this mixed liquid was added 0.27 g of a boron trifluoride-diethyl ether complex, then, a solution composed of 0.18 g of trimethylsilyl cyanide and 2 ml of methyl tert-butyl ether was dropped at -80 to -70°C. After stirring for 4 hours at the same temperature, 5.3 g of a 7% sodium hydrogen carbonate aqueous solution was added and the mixture was heated up to 25°C before liquid-partitioning. The aqueous layer was again subjected to an extraction and liquid-partitioning operation using 10 ml of methyl tert-butyl ether. The resultant organic layers were all mixed, then, sodium sulfate was added and mixed. Next, solid was removed by filtration. The resultant solution was partially concentrated under reduced pressure to distill off the solvent, obtaining 2.17 g of a solution containing 0.10 g (0.52 mmol) of 3-tert-butoxycarbonyl-6,6-dimethyl-3-azabicyclo[3.1.0]hexane-2-carbonitrile.

The yield with respect to 3-tert-butoxycarbonyl-6,6-dimethyl-3-azabicyclo[3.1.0]hexan-2-ol was 59.6%.

### Example 15

### Synthesis Example of (1R,2S,5S)-3-tert-butoxycarbonyl-6,6-dimethyl-3-azabicyclo[3.1.0]hexane-2-carbonitrile

1.97 g of a boron trifluoride-diethyl ether complex and 7.5 g of toluene were mixed and cooled down to -40°C. Into this cooled liquid, a solution prepared by mixing 6.0 g of a toluene solution containing 1.50 g (6.6 mmol) of (1R,5S)-3-tert-butoxycarbonyl-6,6-dimethyl-3-azabicyclo[3.1.0]hexan-2-ol obtained in the same manner as in Example 1, 1.37 g of trimethylsilyl cyanide and 2.5 ml of toluene was dropped at -40°C over a period of 7 hours, then, the mixture was thermally insulated at the same temperature for 1 hour. The resultant solution was dropped at 0 to 10°C into 7.5 g of water cooled to 0 to 10°C, then, 3.5 g of a 40% sodium hydroxide aqueous solution was added, further, an 8% sodium hypochlorite solution was added, then, the mixture was stirred for 1 hour at room temperature. The residual cyan in the solution was measured using a test kit for detecting free cyan (KYORITSU CHEMICAL-CHECK Lab., Corp.: type WAK-CN), to find no residual cyan.

To the solution after stirring was added sulfuric acid to adjust pH to 7 to 8 before performing liquid-partitioning. The resultant organic layer was washed with 7.5 g of water, to obtain 20.5 g of a solution containing 1.34 g (5.7 mmol) of (1R,2S,5S)-3-tert-butoxycarbonyl-6,6-dimethyl-3-azabicyclo[3.1.0]hexane-2-carbonitrile.

The yield with respect to (1R,5S)-3-tert-butoxycarbonyl-6,6-dimethyl-3-azabicyclo[3.1.0]hexan-2-ol was 85.7%.

### Example 16

### Synthesis Example of methyl (1R,2S,5S)-3-tert-butoxycarbonyl-6,6-dimethyl-3-azabicyclo[3.1.0]hexane-2-carboimidate and methyl (1R,2S,5S)-3-tert-butoxycarbonyl-6,6-dimethyl-3-azabicyclo[3.1.0]hexane-2-carboxylate

56.0 g of a solution containing 36.5 g (154 mmol) of (1R,2S,5S)-3-tert-butoxycarbonyl-6,6-dimethyl-3-azabicyclo[3.1.0]hexane-2-carbonitrile obtained in the same manner as in Example 9, and 349 g of methanol and 53.4 g of potassium carbonate were mixed, then, cooled down to 0°C, and stirred for 9 hours at 0°C.

A solution containing 40.0 g (149 mmol) of methyl (1R,2S,5S)-3-tert-butoxycarbonyl-6,6-dimethyl-3-azabicyclo[3.1.0]hexane-2-carboimidate was obtained.

The yield with respect to (1R,2S,5S)-3-tert-butoxycarbonyl-6,6-dimethyl-3-azabicyclo[3.1.0]hexane-2-carbonitrile was 96.5%.

Into a solution containing 40.0 g (149 mmol) of methyl (1R,2S,5S)-3-tert-butoxycarbonyl-6,6-dimethyl-3-azabicyclo[3.1.0]hexane-2-carboimidate described above, 491 g of 7% hydrochloric acid was dropped at 0°C over a period of 1 hour, then, the mixture was stirred at 0°C for 8 hours, then, concentrated under reduced pressure to distill off the solvent, then, 182 g of methyl tert-butyl ether was added to cause liquid-partitioning. The aqueous layer was again subjected to an extraction and liquid-partitioning operation using 182 g of methyl tert-butyl ether. The resultant organic layers were all mixed, then, 109 g of water was added and mixed to cause liquid-partitioning. The resultant organic layer was concentrated under reduced pressure to distill off the solvent, then, 109 g of toluene was further added, then, concentrated under reduced pressure to distill off the solvent, obtaining 75.0 g of a solution containing 40.6 g (151 mol) of methyl (1R,2S,5S)-3-tert-butoxycarbonyl-6,6-dimethyl-3-azabicyclo[3.1.0]hexane-2-carboxylate.

The yield with respect to (1R,2S,5S)-3-tert-butoxycarbonyl-6,6-dimethyl-3-azabicyclo[3.1.0]hexane-2-carbonitrile was 97.3%.

### Example 17

### Synthesis Example of ethyl 3-tert-butoxycarbonyl-6,6-dimethyl-3-azabicyclo[3.1.0]hexane-2-carboxylate

1.35 g of a solution containing 1.00 g (4.2 mmol) of 3-tert-butoxycarbonyl-6,6-dimethyl-3-azabicyclo[3.1.0]hexane-2-carbonitrile, and 25 ml of ethanol and 1.46 g of potassium carbonate were stirred at 20 to 30°C for 70 hours, then, 13.5 g of 7% hydrochloric acid was dropped at 20 to 30°C, then, the mixture was stirred for 3 hours. By concentration under reduced pressure, the solvent was distilled off, then, 10 ml of methyl tert-butyl ether and 10 ml of water were added and mixed before liquid-partitioning. Next, an operation of extraction of an aqueous layer with 10 ml of methyl tert-butyl ether and liquid-partitioning thereof was repeated twice. The resultant organic layers were all mixed, then, sodium sulfate was added into this organic layer and mixed, then, solid was removed by filtration. The resultant solution was concentrated under reduced pressure to distill off the solvent, obtaining a solution containing 1.38 g of ethyl 3-tert-butoxycarbonyl-6,6-dimethyl-3-azabicyclo[3.1.0]hexane-2-carboxylate. In this solution, about 10% of 3-tert-butoxycarbonyl-6,6-dimethyl-3-azabicyclo[3.1.0]hexane-2-carbonitrile as a raw material compound remained.

Into the resultant solution, 25 ml of ethanol and 1.46 g of potassium carbonate were again added, and the mixture was stirred at 20 to 30°C for 17 hours, then, 13.5 g of 7% hydrochloric acid was dropped at 20 to 30°C, then, the mixture was stirred for 2 hours. By concentration under reduced pressure, the solvent was distilled off, then, 10 ml of methyl tert-butyl ether and 10 ml of water were added and mixed before liquid-partitioning. Further, an aqueous layer extraction and liquid-partitioning operation was carried out twice using 10 ml of methyl tert-butyl ether. The resultant organic layers were all mixed, then, into this organic layer was added sodium sulfate and mixed. Next, solid was filtrated, then, the resultant filtrate was concentrated under reduced pressure to distill off the solvent. After distillation, 1.34 g of a solution containing 1.18 g (4.2 mmol) of ethyl 3-tert-butoxycarbonyl-6,6-dimethyl-3-azabicyclo[3.1.0]hexane-2-carboxylate was obtained.

The yield of 3-tert-butoxycarbonyl-6,6-dimethyl-3-azabicyclo[3.1.0]hexane-2-carboxylic acid ethyl ester with respect to 3-tert-butoxycarbonyl-6,6-dimethyl-3-azabicyclo[3.1.0]hexane-2-carbonitrile was 98.6%.

### Example 18

### Synthesis Example of methyl (1R,2S,5S)-3-tert-butoxycarbonyl-6,6-dimethyl-3-azabicyclo[3.1.0]hexane-2-carboimidate

1.42 g of a solution containing 1.00 g (4.23 mmol) of (1R,2S,5S)-3-tert-butoxycarbonyl-6,6-dimethyl-3-azabicyclo[3.1.0]hexane-2-carbonitrile obtained in the same manner as in Example 9, and 5.0 g of methanol and 1.47 g of potassium carbonate were mixed and cooled down to 0°C, and stirred for 20 hours at 0°C. To this solution was added 5.0 g of water and 5.0 g of toluene at 0°C and a liquid-partitioning operation was performed. The aqueous layer was again subjected to an extraction and liquid-partitioning operation using 2.5 g of toluene. The resultant organic layers were all mixed, and the solvent was distilled off by concentration under reduced pressure, obtaining 1.15 g of an oily substance containing 1.06 g (3.95 mol) of (1R,2S,5S)-3-tert-butoxycarbonyl-6,6-dimethyl-3-azabicyclo[3.1.0]hexane-2-carboimidate. The yield of methyl (1R,2S,5S)-3-tert-butoxycarbonyl-6,6-dimethyl-3-azabicyclo[3.1.0]hexane-2-carboimidate with respect to (1R,2S,5S)-3-tert-butoxycarbonyl-6,6-dimethyl-3-azabicyclo[3.1.0]hexane-2-carbonitrile was 93.4%.

The results of ¹H-NMR (CDCl₃) of methyl (1R,2S,5S)-3-tert-butoxycarbonyl-6,6-dimethyl-3-azabicyclo[3.1.0]hexane-2-carboimidate are shown below.

δ = 4.14 to 4.01 d (1H), 3.81 s (3H), 3.64 to 3.43 m (2H), 1.51 to 1.28 m (2H), 1.39 (9H), 1.03 s (3H), 0.93 s (3H)

### Example 19

### Synthesis Example of methyl (1R,2S,5S)-6,6-dimethyl-3-azabicyclo[3.1.0]hexane-2-carboxylate hydrochloride

Into a solution prepared by dissolving 1.80 kg of a hydrogen chloride gas in 6.82 kg of methanol, 9.13 kg of a toluene solution containing 6.68 kg (24.8 mol) of methyl (1R,2S,5S)-3-tert-butoxycarbonyl-6,6-dimethyl-3-azabicyclo[3.1.0]hexane-2-carboxylate obtained in the same manner as in Example 16 was dropped at 25 to 30°C over a period of 3 hours, and 4.2 kg of toluene was added and the mixture was stirred at 25 to 30°C for 4 hours. 28.0 kg of methyl tert-butyl ether was added, and a seed crystal of methyl (1R,2S,5S)-6,6-dimethyl-3-azabicyclo[3.1.0]hexane-2-carboxylate hydrochloride was added at 30°C. Subsequently, 78.7 kg of methyl tert-butyl ether was dropped over a period of 2 hours, then, the mixture was cooled down to - 5°C over a period of 11 hours, and stirred at -5°C for 3 hours. The deposited crystal was filtrated, then, washing with 13.3 kg of methyl tert-butyl ether was carried out three times. 4.94 kg of the resultant crystal was dried under reduced pressure, obtaining 4.47 kg (21.7 mol) of a crystal containing methyl (1R,2S,5S)-6,6-dimethyl-3-azabicyclo[3.1.0]hexane-2-carboxylate hydrochloride.

The yield of methyl (1R,2S,5S)-6,6-dimethyl-3-azabicyclo[3.1.0]hexane-2-carboxylate hydrochloride with respect to methyl (1R,2S,5S)-3-tert-butoxycarbonyl-6,6-dimethyl-3-azabicyclo[3.1.0]hexane-2-carboxylate was 91.0%.

Next, 79.6 g of the resultant crystal [containing 72.0 g of methyl (1R,2S,5S)-6,6-dimethyl-3-azabicyclo[3.1.0]hexane-2-carboxylate hydrochloride] and 274 g of 2-propanol were mixed and heated up to 45 to 50°C to dissolve the crystal. To this solution was added a seed crystal of methyl (1R,2S,5S)-6,6-dimethyl-3-azabicyclo[3.1.0]hexane-2-carboxylate hydrochloride at 38°C. After stirring for 30 minutes at 38°C, the mixture was cooled down to -5°C over a period of 9 hours. After stirring at -5°C for 1 hour, 274 g of methyl tert-butyl ether was dropped over a period of 3 hours, and further, the mixture was thermally insulated at -5°C. The deposited crystal was filtrated, then, washed twice with a solution composed of 36 g of 2-propanol and 36 g of methyl tert-butyl ether, and further washed using 72 g of methyl tert-butyl ether. The resultant crystal was dried under reduced pressure, obtaining 64.2 g of a crystal of methyl (1R,2S,5S)-6,6-dimethyl-3-azabicyclo[3.1.0]hexane-2-carboxylate hydrochloride with a yield in the re-crystallization operation of 89.2%.

The overall yield from methyl (1R,2S,5S)-3-tert-butoxycarbonyl-6,6-dimethyl-3-azabicyclo[3.1.0]hexane-2-carboxylate was 81.2%.

### Example 20

### Synthesis Example of methyl (1R,2S,5S)-6,6-dimethyl-3-azabicyclo[3.1.0]hexane-2-carboxylate hydrochloride

Into 173 g of a methanol solution containing 20 wt% of hydrogen chloride, a solution prepared by mixing 152 g of a toluene solution containing 86.3 g (321 mmol) of methyl (1R,2S,5S)-3-tert-butoxycarbonyl-6,6-dimethyl-3-azabicyclo[3.1.0]hexane-2-carboxylate obtained in the same manner as in Example 16, and 12.0 g of toluene was dropped at 20°C. 8.6 g of toluene was added and the mixture was stirred at 20°C for 5 hours. To this solution was added 1036 g of methyl tert-butyl ether, then, a seed crystal of methyl (1R,2S,5S)-6,6-dimethyl-3-azabicyclo[3.1.0]hexane-2-carboxylate hydrochloride was added, and further, 690 g of methyl tert-butyl ether was dropped over a period of 1 hour. The resultant mixed liquid was cooled down to 0°C over a period of 2 hours. After stirring for 2 hours at 0°C, the deposited crystal was filtrated, then, washed using 210 g of methyl tert-butyl ether. The resultant crystal was dried under reduced pressure to obtain 53.8 g of a crystal of methyl (1R,2S,5S)-6,6-dimethyl-3-azabicyclo[3.1.0]hexane-2-carboxylate hydrochloride.

The yield of methyl (1R,2S,5S)-6,6-dimethyl-3-azabicyclo[3.1.0]hexane-2-carboxylate hydrochloride with respect to methyl (1R,2S,5S)-3-tert-butoxycarbonyl-6,6-dimethyl-3-azabicyclo[3.1.0]hexane-2-carboxylate was 81.6%.

### Example 21

### Synthesis Example of methyl 6,6-dimethyl-3-azabicyclo[3.1.0]hexane-2-carboxylate hydrochloride

0.32 g (1.2 mmol) of methyl 3-tert-butoxycarbonyl-6,6-dimethyl-3-azabicyclo[3.1.0]hexane-2-carboxylate, and 1.16 g of an ethyl acetate solution containing 15 wt% of hydrogen chloride were mixed at 25°C, then, thermally insulated at 25°C.

Disappearance of methyl 3-tert-butoxycarbonyl-6,6-dimethyl-3-azabicyclo[3.1.0]hexane-2-carboxylate was confirmed, then, the reaction liquid was concentrated under reduced pressure to distill off the solvent. To the resultant concentration residue was added 0.32 g of toluene and 0.5 ml of methanol, and the mixture was heated up to 40°C to dissolve the deposited crystal. Into the resultant solution, 1.6 ml of methyl tert-butyl ether was dropped. A slurry containing the deposited crystal was cooled down to 0°C. The slurry liquid was filtrated to obtain a crystal which was then washed using methyl tert-butyl ether. The resultant crystal was dried under reduced pressure, to obtain 0.11 g (0.53 mmol) of methyl 6,6-dimethyl-3-azabicyclo[3.1.0]hexane-2-carboxylate hydrochloride.

The yield of methyl 6,6-dimethyl-3-azabicyclo[3.1.0]hexane-2-carboxylate hydrochloride was 44.9% with respect to methyl 3-tert-butoxycarbonyl-6,6-dimethyl-3-azabicyclo[3.1.0]hexane-2-carboxylate.

### Example 22

### Synthesis Example of methyl (1R,2S,5S)-6,6-dimethyl-3-azabicyclo[3.1.0]hexane-2-carboxylate hydrochloride

1.00 g (4.23 mmol) of (1R,2S,5S)-3-tert-butoxycarbonyl-6,6-dimethyl-3-azabicyclo[3.1.0]hexane-2-carbonitrile, and 7.00 g of a methanol solution containing 20 wt% of hydrogen chloride were mixed at 20°C to 30°C, then, thermally insulated at the same temperature. Disappearance of the raw material compounds was confirmed, then, the reaction liquid was concentrated under reduced pressure to distill off the solvent, to obtain 1.06 g of an oily substance containing 0.55 g (2.67 mmol) of methyl (1R,2S,5S)-6,6-dimethyl-3-azabicyclo[3.1.0]hexane-2-carboxylate hydrochloride.

The yield of methyl (1R,2S,5S)-6,6-dimethyl-3-azabicyclo[3.1.0]hexane-2-carboxylate hydrochloride with respect to (1R,2S,5S)-3-tert-butoxycarbonyl-6,6-dimethyl-3-azabicyclo[3.1.0]hexane-2-carbonitrile was 63.1%.

### Industrial Applicability

The proline derivative obtained by the production method of the present invention is useful as a chemical raw material and medical-agricultural drug intermediate.

## Claims

1. A method of producing a proline derivative of the following formula (1) or an acid addition salt thereof, comprising the following four steps A to D: 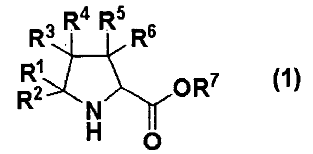 [wherein, any two of R¹, R², R³, R⁴, R⁵ and R⁶ are connected to form an optionally substituted polymethylene group having 1 to 4 carbon atoms, one or no-mutually-adjacent two methylene groups constituting the polymethylene group may be substituted by an oxygen atom, one or two ethylene groups constituting the polymethylene group may be substituted by a vinylene group, no-mutually adjacent two methylene groups constituting the polymethylene group may be mutually connected via an oxygen atom, methylene group, ethylene group or vinylene group,
R¹, R², R³, R⁴, R⁵ and R⁶ not constituting the above-described polymethylene group represent each independently a hydrogen atom, halogen atom, cyano group, optionally substituted linear, branched or cyclic alkyl group having 1 to 10 carbon atoms, optionally substituted linear, branched or cyclic alkenyl group having 2 to 10 carbon atoms, optionally substituted aryl group having 6 to 20 carbon atoms, optionally substituted amino group, -ORₐ group or - SR_{b} group, Rₐ and R_{b} represent each independently a hydrogen atom, alkylcarbonyl group having 2 to 10 carbon atoms, arylcarbonyl group having 7 to 20 carbon atoms, aralkyl group having 7 to 20 carbon atoms, alkoxyalkyl group having 2 to 10 carbon atoms, trialkylsilyl group having 3 to 10 carbon atoms, alkyl group having 1 to 10 carbon atoms or aryl group having 6 to 20 carbon atoms,
R⁷ represents an optionally substituted linear alkyl group having 1 to 10 carbon atoms, branched alkyl group having 2 to 10 carbon atoms, linear alkenyl group having 2 to 10 carbon atoms, branched alkenyl group having 3 to 10 carbon atoms or aralkyl group having 7 to 20 carbon atoms.]
(Step A)
A step of reacting N-protected pyrrolidinones of the following formula (2) with a reducing agent, to produce N-protected pyrrolidinols of the following formula (3): 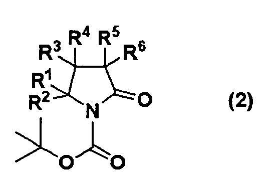 [wherein, R¹, R², R³, R⁴, R⁵ and R⁶ represent the same meanings as described above.] 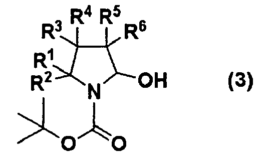 [wherein, R¹, R², R³, R⁴, R⁵ and R⁶ represent the same meanings as described above.]
(Step B)
A step of reacting the N-protected pyrrolidinols of the formula (3) obtained in the step A with a cyanodizing agent, to produce N-protected cyanopyrrolidines of the following formula (4): 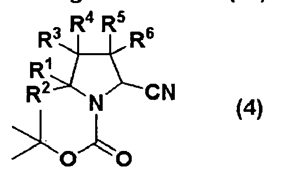 [wherein, R¹, R², R³, R⁴, R⁵ and R⁶ represent the same meanings as described above.]
(Step C)
A step of reacting the N-protected cyanopyrrolidines of the formula (4) obtained in the step B with alcohols and a base, to obtain imidates of the following formula (5), and treating the imidates with an acid, to produce N-protected prolines of the following formula (6): 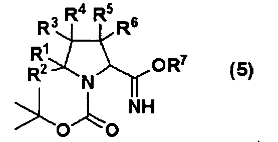 [wherein, R¹, R², R³, R⁴, R⁵, R⁶ and R⁷ represent the same meanings as described above.] 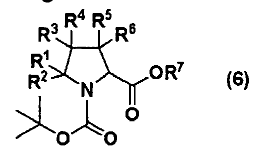 [wherein, R¹, R², R³, R⁴, R⁵, R⁶ and R⁷ represent the same meanings as described above.]
(Step D)
A step of treating the N-protected prolines of the formula (6) obtained in the step C with an acid, to produce a proline derivative of the above-described formula (1) or an acid addition salt thereof.

2. A method of producing a proline derivative of the following formula (1) or an acid addition salt thereof, comprising the following three steps A, B and E: 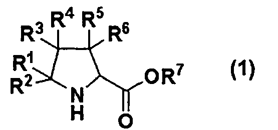 [wherein, R¹, R², R³, R⁴, R⁵, R⁶ and R⁷ represent the same meanings as described above.]
(Step A)
A step of reacting N-protected pyrrolidinones of the following formula (2) with a reducing agent, to produce N-protected pyrrolidinols of the following formula (3): 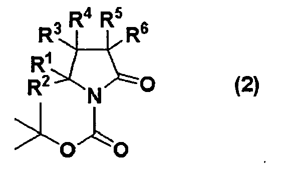 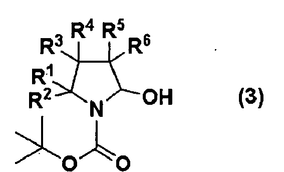 [in the formulae (2) and (3), R¹, R², R³, R⁴, R⁵ and R⁶ represent the same meanings as described above.]
(Step B)
A step of reacting the N-protected pyrrolidinols of the formula (3) obtained in the step A with a cyanodizing agent, to produce N-protected cyanopyrrolidines of the following formula (4): 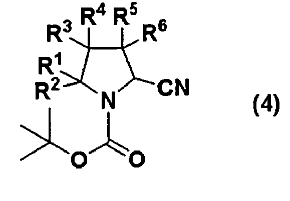 [wherein, R¹, R², R³, R⁴, R⁵ and R⁶ represent the same meanings as described above.]
(Step E)
A step of reacting the N-protected cyanopyrrolidines of the formula (4) obtained in the Step B with alcohols and an acid, to produce a proline derivative of the formula (1) or an acid addition salt thereof.

3. The production method according to Claim 1 or 2, wherein R¹ and R² represent a hydrogen atom.

4. The production method according to any one of Claims 1 to 3, wherein R³ and R⁵ represent a hydrogen atom.

5. The production method according to any one of Claims 1 to 4, wherein R⁴ and R⁶ are connected to form an optionally substituted polymethylene group having 1 to 4 carbon atoms.

6. The production method according to any one of Claims 1 to 5, wherein R⁴ and R⁶ are connected to form a (CH₃)₂C< group.

7. The production method according to any one of Claims 1 to 6, wherein R⁷ is an alkyl group having 1 to 4 carbon atoms.

8. The production method according to any one of Claims 1 to 7, wherein the reducing agent to be used in the step A is lithium triethylborohydride or diisobutylaluminum hydride.

9. The production method according to any one of Claims 1 to 8, wherein the cyanodizing agent to be used in the step B is trimethylsilyl cyanide.

10. The production method according to Claim 9, wherein the cyanodizing agent to be used in the step B is trimethylsilyl cyanide, and a boron trifluoride complex is used together as an acid catalyst.

11. The production method according to Claim 9 or 10, wherein N-protected pyrrolidinols of the formula (3) and trimethylsilyl cyanide are dropped into a solution containing a boron trifluoride complex.

12. The production method according to any one of Claims 1 to 11, wherein the acid to be used in the step D and the step E is hydrogen chloride.

13. The production method according to any one of Claims 1 to 12, wherein a post-treatment operation is carried out using an oxidizing agent in the step B.

14. N-protected pyrrolidinols of the following formula (3): 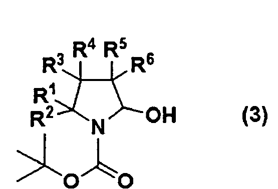 [wherein, R¹, R², R³, R⁴, R⁵ and R⁶ represent the same meanings as described above.].

15. The N-protected pyrrolidinols according to Claim 14, wherein in the formula (3), R¹, R², R³ and R⁵ represent a hydrogen atom, and R⁴ and R⁶ are connected to form an optionally substituted polymethylene group having 1 to 4 carbon atoms.

16. 3-tert-butoxycarbonyl-6,6-dimethyl-3-azabicyclo[3.1.0]hexan-2-ol.

17. (1R,5S)-3-tert-butoxycarbonyl-6,6-dimethyl-3-azabicyclo[3.1.0]hexan-2-ol.

18. N-protected cyanopyrrolidines of the following formula (4): 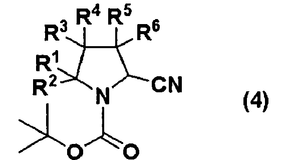 [wherein, R¹, R², R³, R⁴, R⁵ and R⁶ represent the same meanings as described above.].

19. The N-protected cyanopyrrolidines according to Claim 18, wherein in the formula (4), R¹, R², R³ and R⁵ represent a hydrogen atom, and R⁴ and R⁶ are connected to form an optionally substituted polymethylene group having 1 to 4 carbon atoms.

20. 3-tert-butoxycarbonyl-6,6-dimethyl-3-azabicyclo[3.1.0]hexane-2-carbonitrile.

21. (1R,2S,5S)-3-tert-butoxycarbonyl-6,6-dimethyl-3-azabicyclo[3.1.0]hexane-2-carbonitrile.

22. Imidates of the following formula (5): 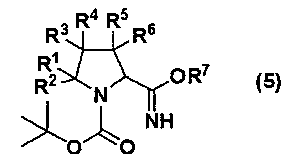 (wherein, R¹, R², R³, R⁴, R⁵, R⁶ and R⁷ represent the same meanings as described above.).

23. The imidates according to Claim 22, wherein in the formula (5), R¹, R², R³ and R⁵ represent a hydrogen atom, R⁴ and R⁶ are connected to form an optionally substituted polymethylene group having 1 to 4 carbon atoms and R⁷ is an alkyl group having 1 to 4 carbon atoms.

24. 3-tert-butoxycarbonyl-6,6-dimethyl-3-azabicyclo[3.1.0]hexane-2-carboimidate.

25. Methyl 3-tert-butoxycarbonyl-6,6-dimethyl-3-azabicyclo[3.1.0]hexane-2-carboimidate.

26. Methyl (1R,2S,5S)-3-tert-butoxycarbonyl-6,6-dimethyl-3-azabicyclo[3.1.0]hexane-2-carboimidate.
